# EUROPEAN PATENT APPLICATION

(11) **EP 1 288 839 A2**
(43) Date of publication of application: **05.03.2003**
(21) Application number: 02019035.1
(22) Date of filing: 27.08.2002
(51) Int. Cl.: G06F 19/00

(54) **Endoscopic image filing system**

(30) Priority: 29.08.2001 JP 2001260079
(71) Applicant: Olympus Optical Co., Ltd., Tokyo (JP)
(72) Inventor: Watai, Makoto, New York 11768 (US); Shibata, Hiroyuki, Yokohama-shi, Kanagawa (US)
(74) Representative: von Hellfeld, Axel, Dr. Dipl.-Phys.

(57) **Abstract**

The present invention adds, to an endoscopic image filing apparatus for filing endoscopic images, patient information, and the like generated at the time of endoscopic examinations performed by means of an endoscope apparatus, a function to manage examination schedules and to newly create endoscopic examination schedules automatically by referring to the schedules thus managed. When, upon setting the endoscopic image filing apparatus to an examination information editing window, a user specifies a patient who is to undergo an examination and designates an examination room and examination date, a schedule for an examination of the patient is automatically created on the basis of schedules already registered, in a time zone not reserved for another examination, whereby user labor can be alleviated and resources such as examination rooms can be utilized effectively.

## Description

### BACKGROUND OF THE INVENTION

### 1. Field of the Invention

The present invention relates to an endoscopic image filing system for recording endoscopic images obtained using an endoscopic image apparatus, and for creating and managing endoscopic examination schedules.

### 2. Description of the Related Art

An endoscopic image apparatus as used conventionally is constituted such that image pickup means are provided in an endoscope which performs observation when a long and narline inserting portion thereof is inserted into an examination site such as a body cavity and constituted such that images of the examination site picked up by the image pickup means, that is, endoscopic images, are displayed on a monitor.

In recent years, endoscopic image filing systems, for which an image filing apparatus for recording endoscopic images is connected with the endoscope apparatus, have been widely used.

An endoscopic image filing system is constituted such that upon pressing an endoscope switch, for example a release switch, with which the endoscope apparatus is provided, still images of endoscopic images displayed on the monitor are recorded by the image filing apparatus.

Further, the endoscopic image filing system is not only capable of recording endoscopic images but is also capable of recording a variety of information relating to an endoscopic examination including a physician's view with regard to recorded endoscopic images, patient information such as the age and gender of the patient, as well as the date and time when the endoscopic examination was conducted.

Further, in a large hospital, a plurality of endoscopic examination rooms are provided for each type of examination, and a multiplicity of endoscopic examinations are conducted in a single day which are divided among a plurality of examiners. A need has thus arisen for endoscopic image filing systems to efficiently manage information for endoscopic examinations that are performed in this plurality of examination rooms.

In addition, when a large number of patients receive examinations, endoscopic image filing systems are also required to suitably manage information on these patients. Such endoscopic image filing systems are not only required to handle endoscopic images but also to manage consumables used in the examinations, and so forth.

Conventionally, it has been necessary to manage information relating to endoscopic examinations separately from information on resources such as endoscopic examination rooms, and users were required to put together a new endoscopic examination schedule after confirming other endoscopic examination schedules and usage states of endoscopic examination rooms.

Japanese Patent Application Laid Open No. 2000-033072 is an example of such prior art.

This prior art example permits information generated using an endoscope apparatus to be obtained by means of a filing apparatus, whereby operability is improved.

However, with this prior art example too, in determining an endoscopic examination schedule as described above, it was necessary to put together a new endoscopic examination schedule after confirming endoscopic examination schedules and usage states of endoscopic examination rooms.

### SUMMARY OF THE INVENTION

An object of the present invention is to provide an endoscopic image filing system whereby endoscopic examination scheduling can be performed automatically, on the basis of conditions and the like required for endoscopic examinations, alleviating user labor and making it possible to utilize resources such as endoscopic examination rooms effectively.

Another object of the present invention is to provide an endoscopic image filing system permitting endoscopic examinations to be conducted smoothly.

The present invention is an endoscopic image filing system comprising: an information input unit operated to input information; an interface section for inputting endoscopic images from an endoscope apparatus and information related with the endoscopic images; processing means for processing information obtained using the information input unit and interface section; managing means for managing schedules for endoscopic examinations performed using the endoscope apparatus; display means for displaying processing information produced by the processing means and management information produced by the managing means, wherein the managing means automatically creates the endoscopic examination schedules by means of at least either conditions preset via the information input unit, or input information inputted via the information input unit when the endoscopic examinations are received, whereby user labor is alleviated and resources such as endoscopic examination rooms can be utilized effectively.

### BRIEF DESCRIPTION OF THE DRAWINGS

Figs. 1 to 22B relate to a first embodiment of the present invention and Fig. 1 shows the overall constitution of the endoscopic image filing system of the first embodiment;
Fig. 2 is a block diagram illustrating the constitution of an endoscope apparatus;
Fig. 3 is a block diagram illustrating the configuration of image filing apparatus hardware;
Fig. 4 is an explanatory drawing that provides an outline for the configuration of windows of the image filing apparatus;
Fig. 5 is a flowchart that provides an overall picture of the flow for operation processing of the image filing apparatus;
Fig. 6 shows an example of the window display of a login window;
Fig. 7 shows an example of the window display of a schedule list window;
Fig. 8 shows an example of the window display of an examination room capacity status display window;
Fig. 9 shows an example of the window display of a calendar window;
Fig. 10 shows an example of the window display of a resource schedule editing window;
Fig. 11 shows an example of the window display of a resource schedule editing window;
Fig. 12 shows an example of the window display of a material management window;
Fig. 13 shows an example of the window display of a patient list window;
Fig. 14 shows an example of the window display of a patient information editing window;
Fig. 15 shows an example of the window display of an examination information editing window;
Fig. 16 shows an example of the window display of a material consumption management window;
Fig. 17 shows an example of the window display of an examination performance window;
Fig. 18 shows an example of the window display of an image selection window;
Fig. 19 shows an example of the window display of a report creation window;
Fig. 20 shows an example of the window display of a report creation window;
Fig. 21 is a flowchart showing the processing flow of processing to set an examination schedule automatically;
Figs. 22A and 22B respectively show usage states of examination rooms and respective availability of examiners for performing examinations on an examination date specified in resource schedules;
Figs. 23A and 23B relate to a second embodiment of the present invention; Fig. 23A is a flowchart showing the processing flow for setting an examination schedule automatically; and
Fig. 23B is a flowchart showing part of the processing flow of processing to set an examination schedule automatically in a modified example.

### DESCRIPTION OF THE PREFERRED EMBODIMENTS

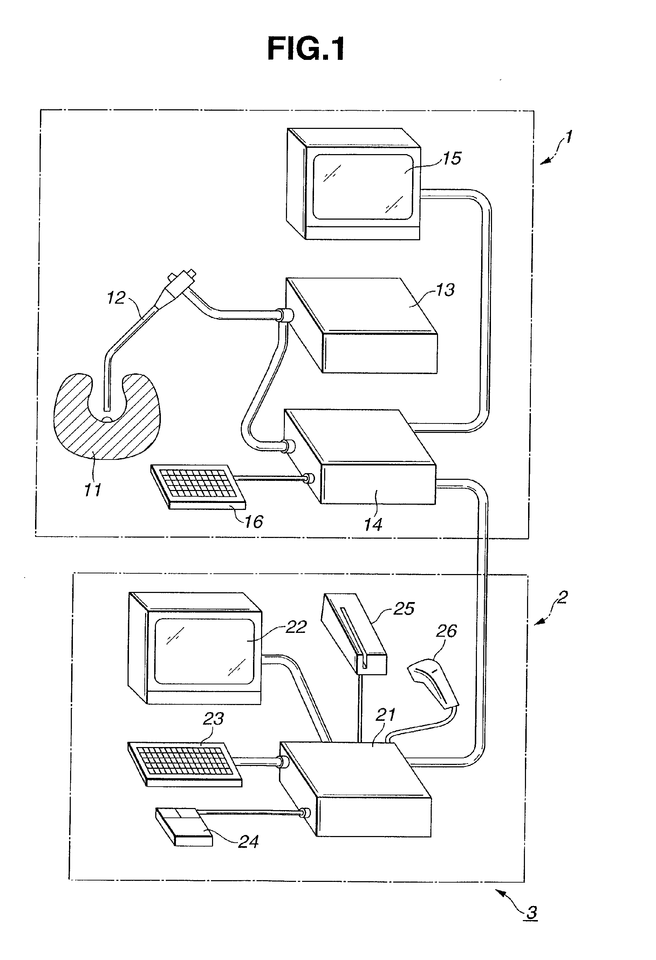

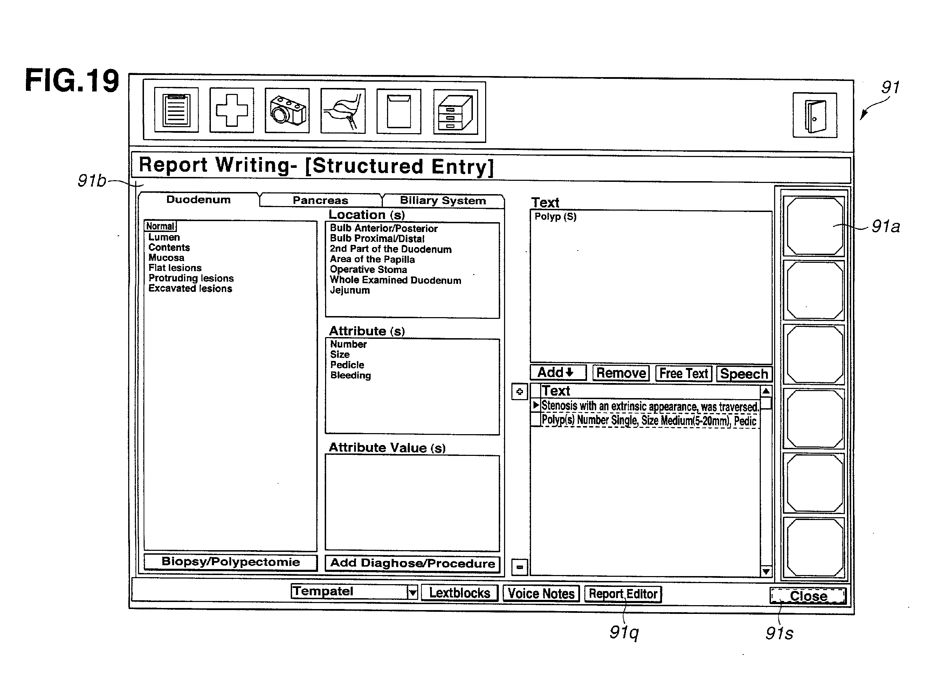

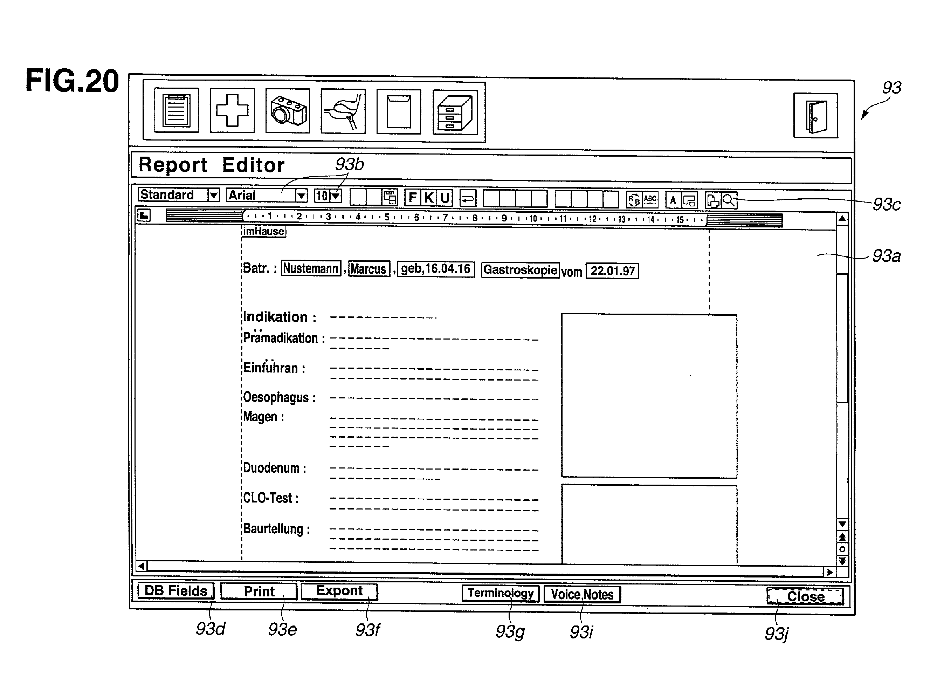

The first embodiment of the present invention will be described hereinbelow by referring to Figs. 1 to 22B.

As shown in Fig. 1, an endoscopic image filing system 3 of the present embodiment principally comprises an endoscope apparatus 1 for performing endoscopic examinations; and an image filing apparatus 2 which is connected with the endoscope apparatus 1 and is for filing images picked up using the endoscope apparatus 1 and the like.

The endoscope apparatus 1 comprises an endoscope 12, which is used in an endoscopic examination by being inserted into an examination site 11; a lighting system 13 for supplying illuminating light to the endoscope 12; an image generating device 14 connected with the endoscope 12 and for performing processing to create an image signal based on signals from an image pickup device; a monitor 15 connected with the image generating device 14 and for displaying images picked up; and a keyboard 16 connected with the image generating device 14 and used for inputting instruction commands and data, and so forth.

Further, the image filing apparatus 2 is connected with the image generating device 14 and comprises a personal computer 21 for performing image filing control and the like; a monitor 22 connected with the personal computer 21 and for displaying a variety of windows; a keyboard 23 for data inputting and the like; a mouse 24 for designating a given predetermined location on a window displayed on the monitor 22; a card reader 25 for inputting patient data or the like by means of a card; and a barcode reader 26 for inputting patient data or the like by means of a bar code.

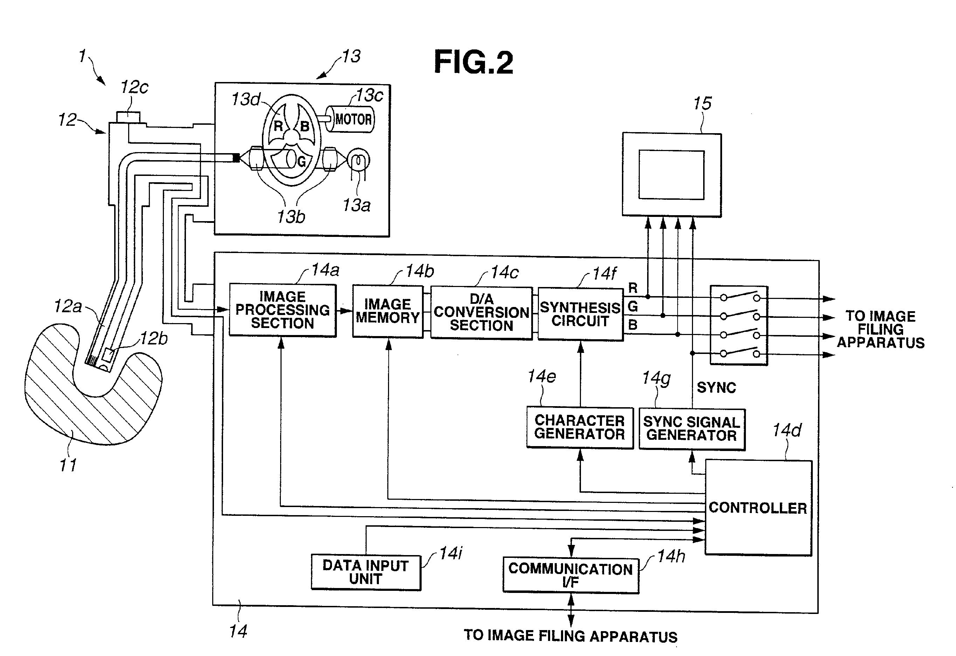

The internal constitution of the endoscope apparatus 1 will be described using Fig. 2.

The endoscope apparatus 1 is provided with a light guide 12a for transmitting illuminating light, which is for illuminating the examination site 11, from the proximal end of the endoscope 12 to the tip of the inserting portion thereof, the proximal end of the light guide 12a being detachably connected to the lighting system 13.

Light emitting means 13a, such as a lamp, that emits illuminating light is provided in the lighting system 13. The illuminating light emitted by the light emitting means 13a is condensed by a condensing optical system 13b and enters the light guide 12a of the endoscope 12.

A rotating plate, which is rotated by a motor 13c and has three optical filters 13d attached thereto which are red, green and blue in color respectively (written as R, G, B respectively in Fig. 2), is disposed at a midway point of the light path of the condensing optical system 13b, whereby the color of the illuminating light illuminating the examination site can be switched using time division.

The illuminating light is transmitted by means of the light guide 12a of the endoscope 12, and exits via the light guide end face, which is attached as an illumination window at the tip of the inserting portion, such that the examination site 11 of the body cavity into which the inserting portion is inserted is illuminated.

An observation window (image pickup window) is provided at the tip of the inserting portion of the endoscope 12 so as to lie adjacent to the illumination window and, in the image formation position of an objective lens which is attached at the observation window, image pickup means 12b for picking up images of the examination site 11 and comprising a solid-state image pickup device such as a charge-coupled device (CCD) for example, are provided.

An image signal of the examination site 11 which is picked up by image pickup means 12b is inputted to an image processing section 14a inside the image generating device 14, and image processing such as color highlighting for example is carried out, such images being temporarily stored in sequence in an image memory 14b.

Image data stored in the image memory 14b is inputted to a D/A conversion section 14c for D/A conversion. The D/A conversion section 14c outputs an RGB format image signal.

The image processing section 14a, image memory 14b, and the like, operate under the control of a controller 14d which is control means for controlling components in the image generating device 14.

The image signal which is output from the D/A conversion section 14c is synthesized by means of a synthesis circuit 14f with an image signal outputted from a character generator 14e described hereinafter.

The RGB format image signal which is output from the synthesis circuit 14f is inputted to the monitor 15 to cause an image of the examination site to be displayed on the monitor 15. At such time, a sync signal (written as SYNC in Fig. 2), which is supplied to the monitor 15, is generated by a sync signal generator 14g that is controlled by the controller 14d.

Character information is inputted by the controller 14d to the character generator 14e such that the character generator 14e is then able to output the character information thus inputted following conversion thereof into an image signal. In other words, character information can then be synthesized and displayed as an image of the examination site in a window of the monitor 15, and it is possible to transmit patient information and other kinds of messages to the user of the endoscope apparatus 1.

The image signal which is output from the synthesis circuit 14f is not only output to the monitor 15 but rather can also be output to the image filing apparatus 2 via a switch circuit so as to also be displayable on the monitor 22 of the image filing apparatus 2. The ON/OFF of the switch circuit is controlled by the controller 14d, for example.

Meanwhile, the endoscope 12 is provided with an endoscope switch 12c which comprises a release switch for inputting an instruction to record images, and a start/end switch which is pushed at the start and end times of the endoscopic examination. The controller 14d detects the state of the endoscope switch 12c so as to be able to perform a control operation corresponding with the operation of this switch.

Further, the controller 14d is also constituted so as to be capable of sending and receiving information to and from the image filing apparatus 2 via a communication interface 14h of the publicly known RS-232C system for example.

The endoscope apparatus 1 is thus capable of transmitting a variety of states of the endoscope apparatus 1 such as the state of the switch 12c to the image filing apparatus 2. The endoscope apparatus 1 is also capable of receiving a variety of messages from the image filing apparatus 2 and displaying received messages on the monitor 15 via the character generator 14a or the like.

Also, a data input unit 14i provided in the image generating device 14 serves for the inputting of data, instructions, and the like, which are inputted via the keyboard 16, to the controller 14d.

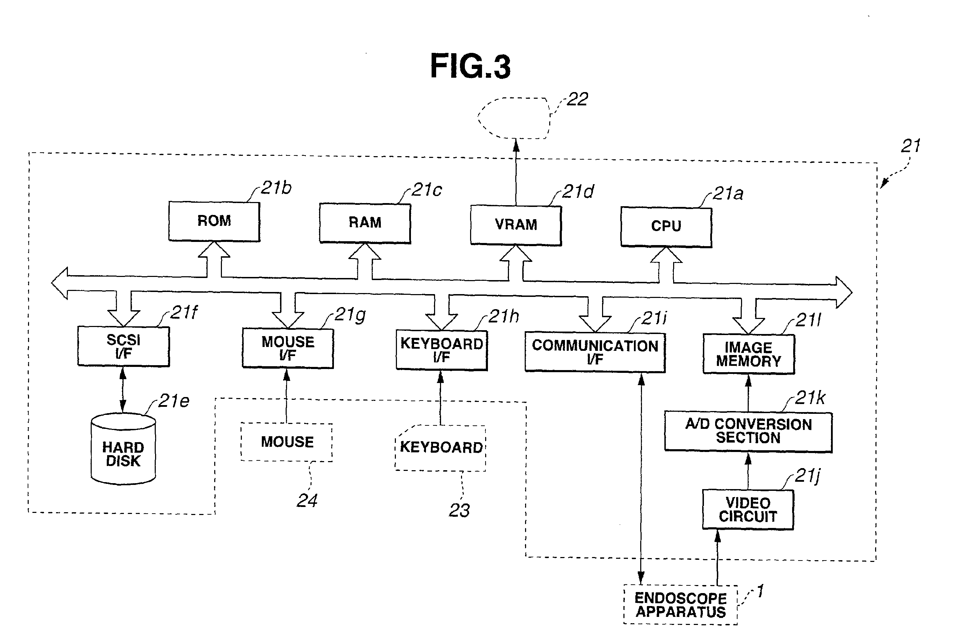

The internal constitution of the personal computer 21 that constitutes the principal component of the image filing apparatus 2 will now be described, referring to Fig. 3.

The personal computer 21 comprises: a CPU 21a, which is principal control means for controlling the components of the personal computer 21; a ROM 21b, in which programs which cause the CPU 21a to operate and/or messages or similar to be displayed on the monitor 22 are stored; a RAM 21c, which is used as the work area of the CPU 21a and as a buffer for a variety of data; a VRAM 21d for temporarily storing image data which is output to the monitor 22; a hard disk 21e for storing image data and a variety of data; an SCSI interface 21f for sending and receiving data to and from the hard disk 21e using the publicly known SCSI system; a mouse interface 21g permitting inputs via the mouse 24; a keyboard interface 21h permitting inputs via the keyboard 23; a communication interface 21i which uses the publicly known RS-232C system, for example, for sending and receiving a variety of data to and from the endoscope apparatus 1; a video circuit 21j, which is an interface circuit for inputting an image signal which is output from the endoscope apparatus 1; an A/D conversion section 21k for A/D converting an image signal which is inputted by the video circuit 21j; and an image memory 211 for temporarily storing image data which is output from the an A/D conversion section 21k, and the like.

By means of the foregoing components, the image filing apparatus 2 whose principal component is the personal computer 21 is capable of displaying image data obtained by the endoscope apparatus 1 on the monitor 22, storing this image data on the hard disk 21e, and so forth.

Also, by obtaining the state of the endoscope switch 12c, the image filing apparatus 2 is capable of dividing up processing by the CPU 21a in accordance with the state of the endoscope switch 12c. The endoscope switch 12c thus permits the recording of images and the recording of examination start and end times, and so forth, for example.

Further, messages from the image filing apparatus 2 can be transmitted to the endoscope apparatus 1.

According to the present embodiment, as described hereinbelow, in addition to performing control such as that for recording, to the hard disk 21e, image information of endoscopic images generated by the endoscope apparatus 1 at the time of endoscopic examinations along with patient information related with these endoscopic images, the CPU 21a of the image filing apparatus 2 performs: processing to create examination schedules for performing endoscopic examinations; processing to manage created examination schedules as resource schedules and to create new examination schedules such that these new examination schedules conform with the resource schedules already being managed; and processing to centrally manage the stock status and the like of endoscopy materials to be used in endoscopic examinations or other materials.

Further, image information of endoscopic images as well as patient information related with these endoscopic images, resource schedule information, and information on materials, or similar, are stored on the hard disk 21e and programs which perform the aforementioned processing under the control of the CPU 21a are also stored on the hard disk 21e.

In accordance with the various windows displayed on the monitor 22, the image filing apparatus 2 executes processing such that the user inputs data and instruction information via the keyboard 23, mouse 24 or similar, and such that the CPU 21a controls each component in accordance with inputted data and/or instruction information. That is, the image filing apparatus 2 is thus capable of executing varied processing in accordance with the flow of the windows displayed on the monitor 22.

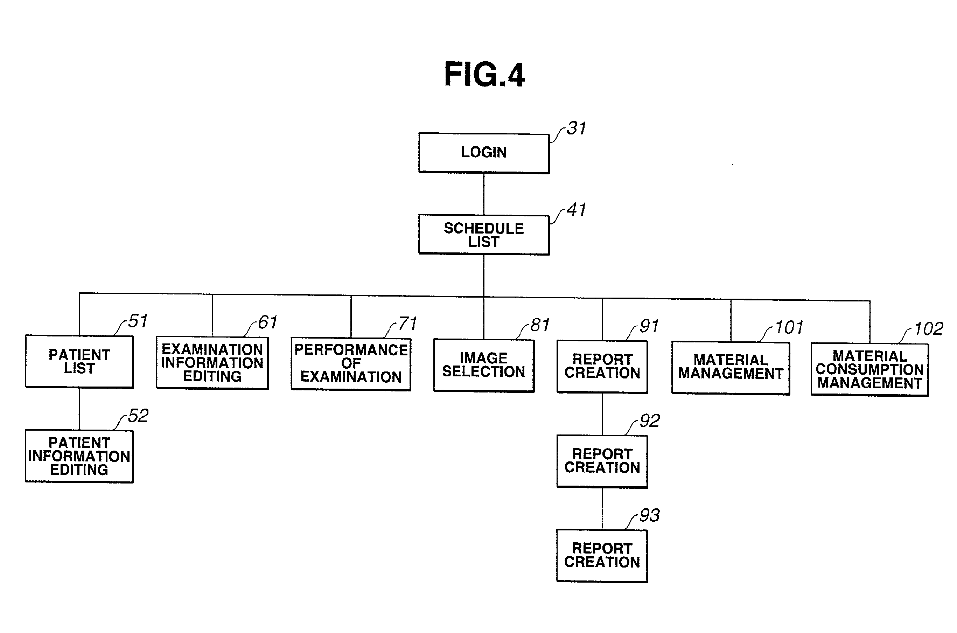

An outline of the configuration of the windows of the image filing apparatus 2 will be explained using Fig. 4.

First, upon startup of the image filing apparatus 2, a login window 31 for user authentication is displayed.

Following the user authentication by means of the login window 31, a schedule list window 41, which is for displaying a list of examination schedules or similar, is displayed.

A patient list window 51 for displaying a list of patient information can be called from the schedule list window 41. A patient information editing window 52 for newly registering patient information or editing patient information which has already been registered can be called from the patient list window 51.

Further, an examination information editing window 61 for reserving an examination by newly registering examination information or editing examination information which has already been registered can be called from the schedule list window 41.

An examination performance window 71 for performing an examination by means of a connection with the endoscope apparatus 1 and capturing images from the endoscope apparatus 1 can be called from the schedule list window 41.

An image selection window 81 for selecting images for creating an examination report, from among captured images, can be called also from the schedule list window 41.

A report creation window 91, which constitutes one window for creating an examination report, can be called also from the schedule list window 41, and a transition can further be made from the report creation window 91 to report creation windows 92, 93 which have different functions.

It is also possible to call a material management window 101 for managing the stock of consumables or other materials, and a material consumption management window 102 for managing materials to be used in examinations, from the schedule list window 41.

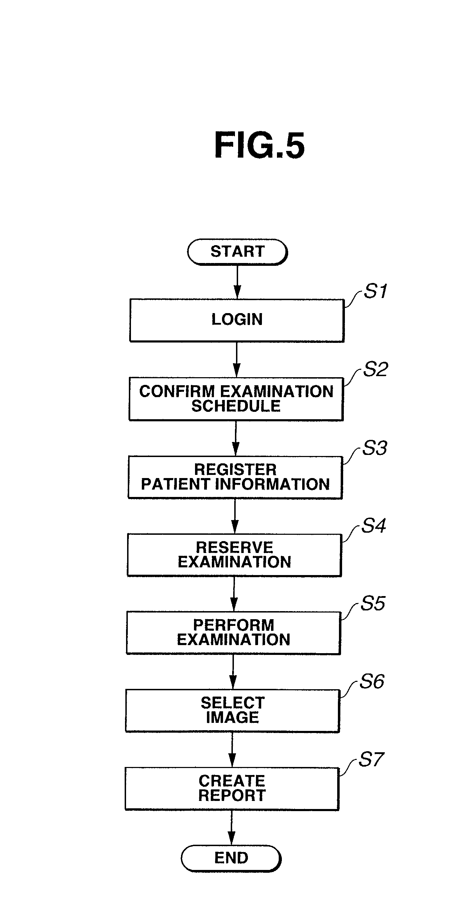

An example of the overall processing flow for operation of the image filing apparatus 2 will now be described by referring to Fig. 5. Reference numerals S1 to S7 in the figure are appended to the processing steps.

First, upon startup of the endoscopic image filing apparatus 2, the login window 31 is displayed, by means of which user authentication is performed. When logging in is performed as indicated in step S1, the schedule list window 41 is displayed at which point an examination schedule is confirmed as shown in step S2.

Thereafter, when, at the time an endoscopic examination is received, the patient to undergo the examination is a new patient, patient information is registered as indicated in step S3 after calling the patient list window 51 and the patient information editing window 52.

Next, a new examination reservation is inputted as indicated in step S4 after calling the examination information editing window 61.

Then, after calling the examination performance window 71, an examination is performed using the endoscope apparatus 1, which is connected with the image filing apparatus 2, as indicated in step S5, and images captured by the endoscope apparatus 1 are stored in the image filing apparatus 2.

Thereafter, upon calling the image selection window 81, images for reference in an examination report creation are selected from among images captured in the course of performing the examination as indicated in step S6.

Next, the report creation windows 91, 92, 93 are called to create an examination report.

The description above is one example of the overall operation processing flow.

The configuration and operation of each of the windows will be described in detail hereinbelow.

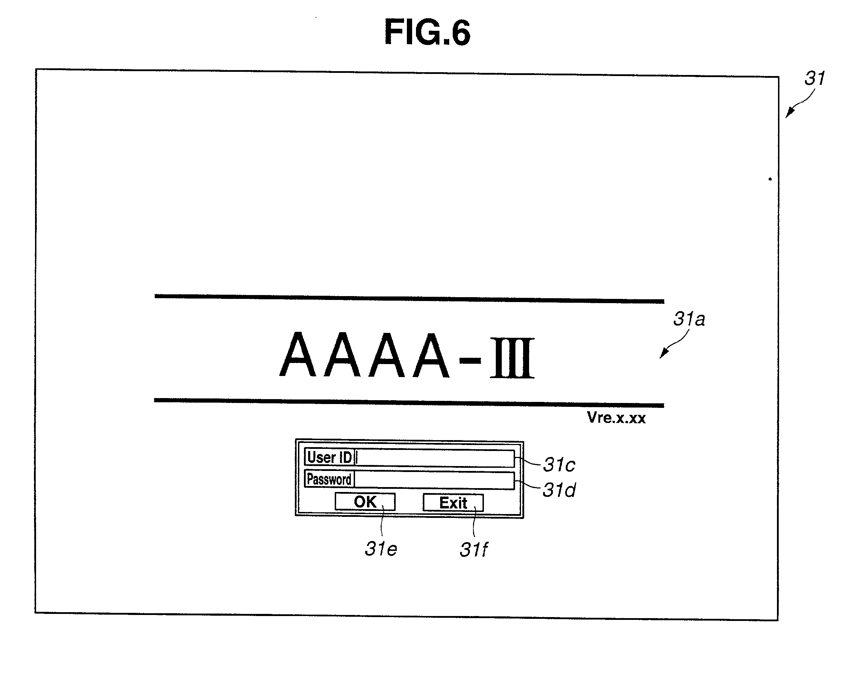

Fig. 6 is an example of the window display of the login window 31.

Upon startup of the image filing apparatus 2, the login window 31 opens.

Note that, in this specification, the viewing of a previously undisplayed window is sometimes expressed as the act of opening a window. Likewise, ending the display of a displayed window is sometimes expressed as an act of closing a window.

A title 31a, which contains the product name of the endoscopic image filing system 3 and corresponding version information or the like, may also be displayed in the login window 31.

Further, in the drawings to which this specification refers, messages or similar in the drawings that illustrate the window displays are displayed in English and partially in German. However, Japanese, or another language and/or another graphics could also be used.

When the login window 31 is displayed, a user ID, which is the ID of the operator, is entered in the input field 31c, and an operator password is entered in the input field 31d, whereupon the operator clicks on an OK button 31e.

Also in this specification, buttons are predetermined regions on a window, and designs, characters, or the like which express the function of these buttons are suitably written in these regions. These regions are appropriately shaded to afford same a three-dimensional quality, to thereby represent the shape of a push button switch on the window.

The operation of clicking on such buttons is an operation in which the cursor of the mouse 24 is moved so as to lie within a button on the window and a button on the mouse 24 is clicked. Clicking on the on-window buttons executes functions which are respectively matched with the buttons.

Here, when authentication is made when the user ID and password thus inputted are valid, a transition is made to the schedule list window 41 which is the next window.

Further, when the user ID and password thus inputted are not valid, the user ID and password must be inputted once again in the login window 31.

Types of authorization for use of the image filing apparatus 2, for example system administrator authorization, physician authorization, and the like, are matched with user IDs such that the functions which may be employed by the image filing apparatus 2 are limited in accordance with these types of usage authorization.

For example, when a user ID of an operator not having system administrator authorization is inputted, this operator cannot make use of functions for maintaining the image filing apparatus 2 which are not described, for example a function for newly registering a user ID or similar.

Also, clicking on an Exit button 31f ends operation of the image filing apparatus 2.

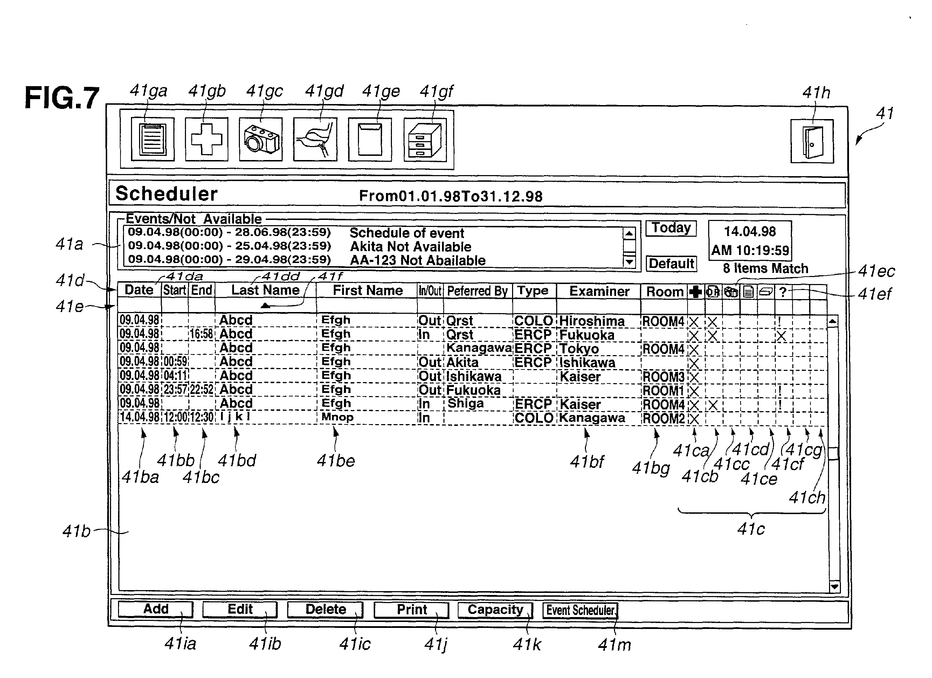

Fig. 7 shows an example of the window display of the schedule list window 41.

A resource schedule list display area 41a for displaying a list of examination resource schedules is disposed in the schedule list window 41.

Further, in this specification, examination resources are intended to indicate people in charge of examinations, namely physicians, nurses and technicians, the examination rooms used for the examinations, and materials employed in the examinations. In addition, information which is contained in one resource schedule is termed a resource schedule record.

One resource schedule record is displayed on one line of the resource schedule list display area 41a.

A resource schedule record displayed on one line of the resource schedule list display area 41a contains a resource usage start date and time and usage end date and time, and the resource name which is the name of the resource.

An examination information list display area 41b for displaying a list of examination information is also disposed in the schedule list window 41.

Also in this specification, one item of examination information is termed an examination information record.

A heading for each data item constituting an examination information record is displayed in the first line of the examination information list display area 41b. The examination information records are displayed from the third line of the examination information list display area 41b, one examination information record being displayed on one line of the examination information list display area 41b.

Data items which constitute the examination information records displayed in the examination information list display area 41b comprise: an examination date 41ba, an examination start time 41bb, an end time 41bc, the patient's last name 41bd, the patient's first name 41be, the examiner in charge of the examination 41bf, and the name of the examination room employed 41bg.

Examination state display fields 41c, which are for displaying states such as the respective state of progress of work relating to the examinations, are also disposed in the examination information list display area 41b.

A classification is made of whether or not all required input items of examination information and all required input items of information on a patient who is to undergo the examination in question have been inputted, and the classification result is indicated in an examination state display field 41ca constituting an examination state display field 41c. For example, when all such information has been inputted, the symbol "X" is displayed in this field, otherwise the field is blank.

A classification is also made of whether or not the date on which an examination consent form is received from the patient has been inputted, and the classification result is indicated in an examination state display field 41cb. For example, when such date has been inputted, the symbol "X" is displayed in this field, and if not inputted, the field is blank.

A classification is also made of whether or not the examination has already been performed, and the classification result is indicated in an examination state display field 41cc. For example, if the examination has already been performed, the symbol "X" is displayed in this field, and if not, the field is blank. Here, an examination which has the symbol "X" in an examination state display field 41cc indicates that images of the examination site 11 of the patient have already been obtained by means of an examination.

A classification is made of whether or not an examination report has been created, and the classification result is indicated in an examination state display field 41cd. For example, when such an examination report has been created, the symbol "X" is displayed in this field, and if not yet created, the field is blank.

A classification is made of whether or not an accounting code has been inputted, and the classification is indicated in an examination state display field 41ce. For example, when such an accounting code has been inputted, the symbol "X" is displayed in this field, and if not inputted, the field is blank.

When a biopsy such as one involving taking a sample of living body tissue is conducted in an endoscopic examination, the state of biopsy examination results is indicated in an examination state display field 41cf. For example, when a biopsy is performed and biopsy examination results have already been obtained, the symbol "X" is displayed in this field; when a biopsy is performed and biopsy examination results have not yet been obtained, the symbol "!" is displayed, and when a biopsy has not been performed, the field is blank.

The state of the patient's insurance at the time of receiving the examination is indicated in examination state display fields 41cg and 41ch. If the insurance type is private, "V" is displayed in an examination state display field 41cg, and if public, "L" is displayed therein. Also, if the form of insurance is that of a member, "M" is displayed in an examination state display field 41ch, and if that of a family member, "F" is displayed therein. The form of insurance is determined in an examination data input operation described hereinafter.

The examination information list display area 41b is not only capable of showing all examination information records but also of filtering the examination information records of interest.

In the first line of the examination information list display area 41b, that is, in the positions of data items in a line in which item names or similar for the data items are displayed, filter buttons 41d, which are buttons for calling respective windows to set filtering conditions for the data items, are disposed.

The filter buttons 41d are buttons that are in a raised state when filtering conditions for corresponding data items are not set.

Buttons in a raised state are on-window buttons which are shaded so that the same appear to have risen and buttons in a sunken state are on-window buttons which are shaded so that the same appear to have sunk.

Clicking on a raised filter button 41d opens a filtering conditions setting window (described hereinafter), which is a window for setting filtering conditions for corresponding data items, whereby it is possible to input filtering conditions. Here, the inputting of filtering conditions causes only those examination information records which satisfy the filtering conditions to be displayed in the examination information list display area 41b.

Filter buttons 41d corresponding with data items for which filtering conditions have been set are buttons in a sunken state.

When there are a plurality of filter buttons 41d in a sunken state, the focusing for the display of examination information records is made by means of an AND condition for filtering conditions which are set by the filter buttons 41d.

Clicking on a sunken filter button 41d cancels filtering conditions which have been set for the corresponding data item and the filter button 41d becomes a raised button.

For example, clicking on a filter button 41da in a position corresponding to the examination date 41ba calls a filtering condition setting window for setting filtering conditions for the examination date 41ba

Further, clicking on a filter button 41dd corresponding with the position of the patient's last name 41bd calls a filtering condition setting window for setting filtering conditions for patient last name.

The examination information list display area 41b shown in Fig. 7 is also able to switch the arranging order of examination information records, designated data items being taken as the key. In positions corresponding with data items in the second line of the examination information list display area 41b, sort buttons 41e for switching the arranging order of examination information records to an ascending or descending order, with data items corresponding with these positions being taken as the key, are disposed.

Upon clicking on the sort button 41e, the arranging order of examination information records can be switched to an ascending order for example, with the data item corresponding with the position of this sort button 41e being taken as the key, and a symbol to indicate a switch to an ascending order, for example an upward facing arrow 41f (▲), is displayed on the sort button 41e. Here, upon clicking on the same sort button 41e once again, the examination information records can be switched to a descending order and the display of the upward facing arrow 41f (▲) changes to that of a downward facing arrow (▼). Thereafter, each time the same sort button 41e is clicked on, the order switches to an ascending order and a descending order respectively.

Also, in the examination information list display area 41b shown in Fig. 7, upon clicking on a filter button 41ec corresponding with an examination state display field 41cc showing whether or not an examination is complete, for example, a filtering condition setting window for setting an indication whether or not the examination is complete as a filtering condition is called.

In addition, in the examination information list display area 41b shown in Fig. 7, upon clicking on a filter button 41ef corresponding with an examination state display field 41cf that shows a classification of the state of biopsy examination results for example, a filtering condition setting window for setting the state of biopsy examination results as a filtering condition is called.

Disposed at the top of the schedule list window 41 shown in Fig. 7 are: a button 41ga which, when clicked, calls the patient information list window 51; a button 41gb which, when clicked, calls the examination information editing window 61; a button 41gc which, when clicked, calls the examination performance window 71; a button 41gd which, when clicked, calls the image selection window 81; a button 41ge which, when clicked, calls the report creation window 91; and a button 41gf which selects and calls a material management window 101 and a material consumption management window 102.

In addition, in a location toward the top right of the schedule list window 41, an Exit button 41h which, when clicked, ends operation of the image filing apparatus 2, is disposed.

Further, disposed at the bottom of the schedule list window 41 are: an Add button 41ia which, when clicked at the time of newly reserving an examination, calls the examination information editing window 61; an Edit button 41ib which, when clicked after selecting an examination information record in the examination information list display area 41b at the time of editing an examination information record already registered, calls the examination information editing window 61; a Delete button 41ic which, when clicked after selecting an examination information record in the examination information list display area 41b, deletes the selected examination information record; a Print button 41j which, when clicked, prints a list of examination information; a button 41k which is clicked when displaying the capacity status of an examination room; and a button 41m which is clicked when newly registering or editing a resource schedule record.

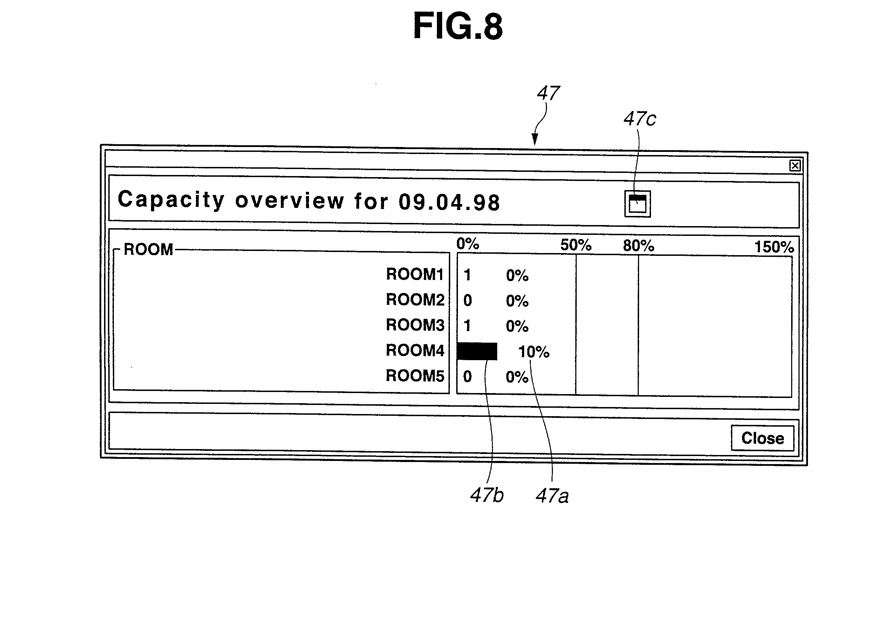

Clicking on the button 41k calls an examination room capacity status display window 47 shown in Fig. 8.

The capacity utilization of each examination room, that is, the proportion of time taken up by reservations in relation to a time interval during which a given examination room is available, is displayed in the examination room capacity status display window 47 in the form of a percentage display 47a and a graph display 47b such as a bar graph.

The graph display 47b is a display using color coding dependent on the capacity utilization grade. For example, color coding is such that the display is red when the capacity utilization of an examination room is equal to or more than 100%, yellow when the capacity utilization is at least 80% and less than 100%, and blue when the capacity utilization is less than 80%, for instance. The relationships between capacity utilization grades and color coding can be optionally set in advance by given means (not shown) and relationships between capacity utilization levels and color coding distinguished by examination room can also be set separately.

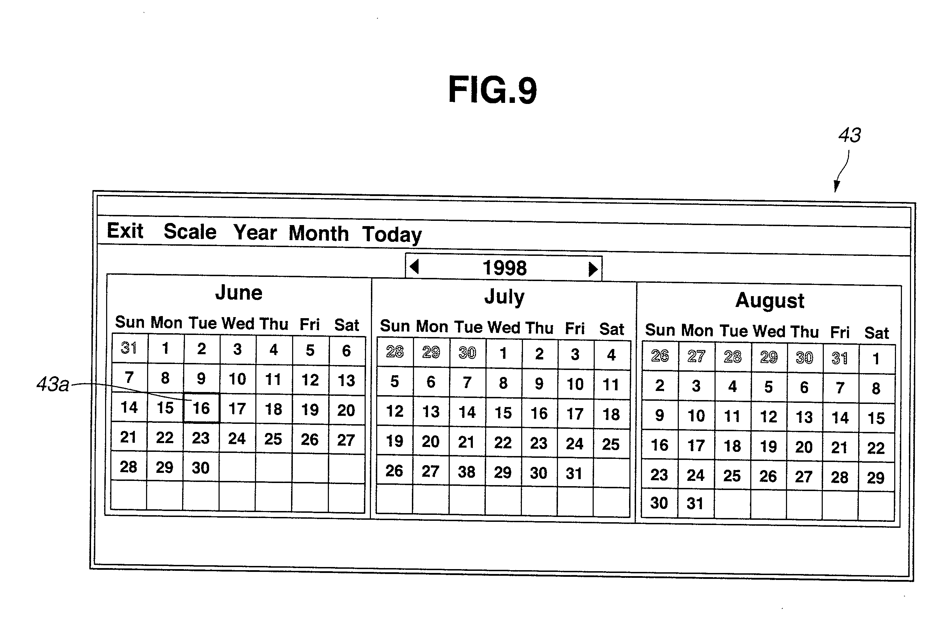

Clicking on a calendar call button 47c calls a calendar window 43 (see Fig. 9), and selecting a date in this calendar window 43 causes the capacity status of each examination room on the selected date to be displayed.

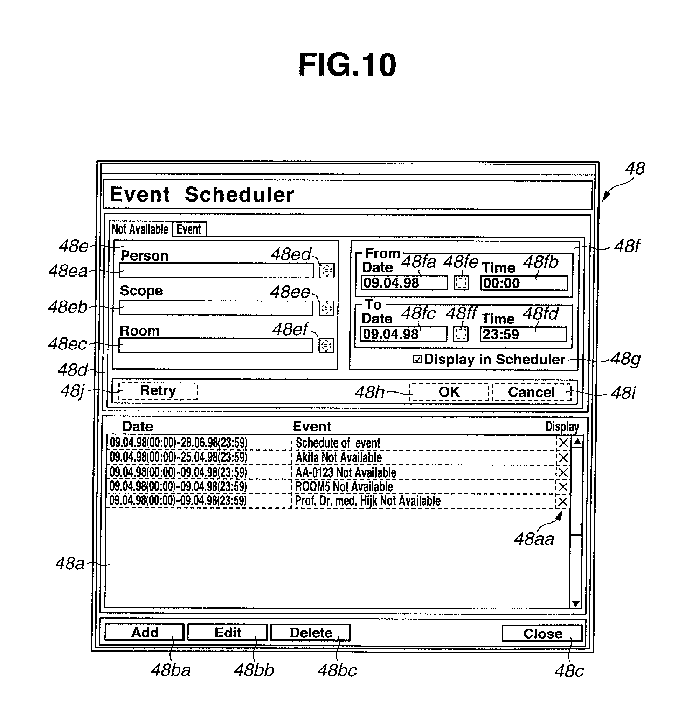

Clicking on the button 41m of the schedule list window 41 shown in Fig. 7 calls a resource schedule editing window 48 as shown in Fig. 10.

A list of resource schedules is displayed in a resource schedule list display area 48a. Data items of resource schedule records displayed in the resource schedule list display area 48a are substantially the same in terms of content as those displayed in the resource schedule list display area 41a of the schedule list window 41 shown in Fig. 7. However, in the resource schedule list display area 48a, display classification fields 48aa are disposed in locations to the right of each resource schedule record, a classification of whether or not each resource schedule record is displayed in the resource schedule list display area 41a of the schedule list window 41 is to be displayed in such fields.

For example, for displaying, the symbol "X" is marked in a given field, and for not displaying, the field is blank. That is, all resource schedule records are displayed in the resource schedule list display area 48a, but only those resource schedule records for which the symbol "X" is marked in a corresponding display classification field 48aa are displayed in the resource schedule list display area 41a of the schedule list window 41.

Disposed at the bottom of the resource schedule editing window 48 are: an Add button 48ba, which is clicked when registering a new resource schedule record; an Edit button 48bb, which is clicked when editing a resource schedule record already registered; a Delete button 48bc which, when clicked after selecting a resource schedule record in the resource schedule list display area 48a, deletes the selected resource schedule record; and a Close button 48c which, when clicked, closes the resource schedule editing window 48 for a return to the schedule list window 41.

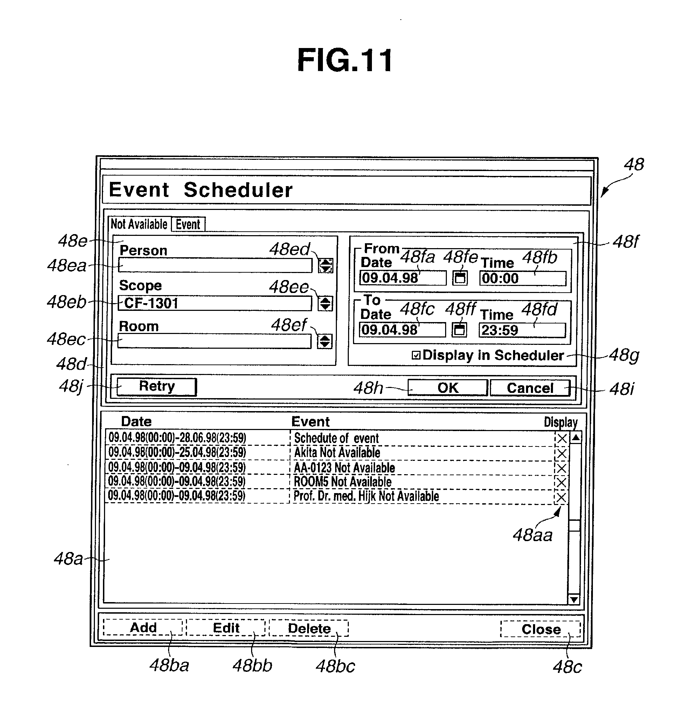

Here, clicking on the Add button 48ba or clicking on the Edit button 48bb after selecting a resource schedule record in the resource schedule list display area 48a renders the state shown in Fig. 11 in which inputs to the resource schedule record editing area 48d used for inputting or editing a resource schedule record become possible. At such time, when editing a resource schedule record which has already been registered, the contents of the already registered resource schedule record are displayed in each field of the resource schedule record editing area 48d.

The resource schedule list display area 48a and resource schedule editing area 48d operate exclusively. In a state in which the resource schedule list display area 48a shown in Fig. 10 is active, buttons disposed within the resource schedule editing area 48d are displayed with a pale color in order to indicate that these buttons are not operational (illustrated using a dotted line frame in the figure). Conversely, in a state in which the resource schedule editing area 48d shown in Fig. 11 is active, buttons disposed within the resource schedule list display area 48a are displayed with a pale color (illustrated using a dotted line frame in the figure).

Fields are disposed within the resource schedule editing area 48d, namely: input fields 48e for inputting the names of examination resources; input fields 48f for inputting a scheduled period for using the examination resources; and a setting field 48g for setting a display classification as to whether or not to display the examination resources in the resource schedule list display area 41a of the schedule list window 41.

Examination resource types comprise the person in charge, the examination equipment (or endoscope) and the examination room, and the input fields 48e comprise an input field 48ea for the name of the person in charge, an input field 48eb for the name of the examination equipment and an input field 48ec for the name of the examination room. When entering information in the input fields 48e, an examination resource name is entered in one of these input fields 48ea, 48eb, and 48ec. Accordingly, the image filing apparatus 2 is able to extract, for example, only those resource schedule records for which examination room usage has been scheduled from among registered resource schedule records, and so forth.

Further, the names of the examination resources entered in the input fields 48e are required input items. The image filing apparatus 2 is constituted such that guidance displays for input fields of required input items are highlighted on the window. For example, required input items are highlighted by displaying guidance displays for input fields of required input items in yellow, but by displaying guidance displays for input fields of input items that are not required input items in black or white or similar.

For example, in a state immediately after clicking on the Add button 48ba, the guidance displays of the input fields 48ea, 48eb, 48ec constituting the input fields 48e ("Person", "Scope", "Room" in the figure) are displayed in yellow.

Here, upon inputting the name of an examination resource in the input field 48ea for example, the input field 48ea is no longer a required input item since inputting has been performed, and the guidance display of the input field 48ea turns white or black. Also, because the input fields 48eb, 48ec also assume a state in which an input is not required, the guidance displays of the input fields 48eb, 48ec also turn white or black.

In locations to the right of the input fields 48ea, 48eb, 48ec, selection list window call buttons 48ed, 48ee, 48ef for calling selection lists corresponding with each of the input fields 48ea, 48eb, 48ec are disposed, it being possible to select names of examination resources from within the selection lists.

Usage period input fields 48f comprise a usage start date input field 48fa, a usage start time input field 48fb, a usage end date input field 48fc, and a usage end time input field 48fd. Calendar call buttons 48fe, 48ff are disposed in locations to the right of the input fields 48fa and 48fc respectively.

In addition, disposed at the bottom of the resource schedule editing area 48d are: an OK button 48h which, whenclicked, confirms information entered in the resource schedule editing area 48d to thereby add or update a resource schedule record; a Cancel button 48i which, when clicked, interrupts editing in the resource schedule editing area 48d to halt the addition or updating of an inputted resource schedule record; and a Retry button 48j which, when clicked, restores the resource schedule editing area 48d to an initial state, that is, to a state immediately after clicking on the Add button 48ba or Edit button 48bb, and renders a state in which inputs to the resource schedule editing area 48d are "retried" from the beginning onwards.

When data is entered in the input fields within the resource schedule editing area 48d and the OK button 48h is clicked, a resource schedule record is newly registered or updated.

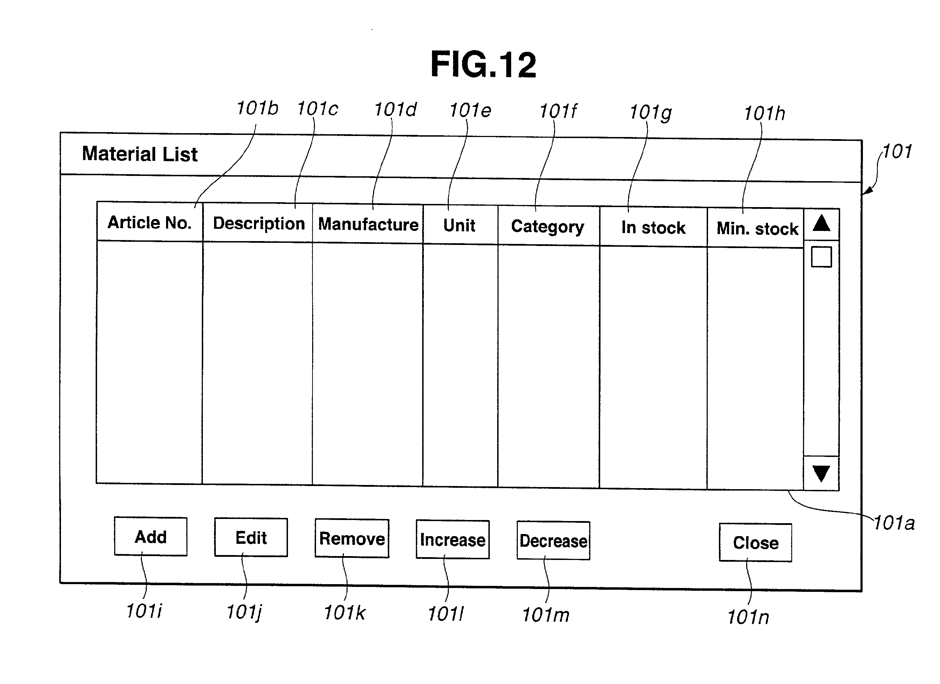

Fig. 12 shows one example of the window display of the material management window 101.

Amaterial list 101a is disposed in the material management window 101. This material list 101a comprises items such as an article number 101b, a description 101c, a manufacturer 101d, a unit 101e indicating an article usage unit, a category 101f indicating categories for whether the article is recyclable or is to be discarded after use, the stock quantity 101g indicating the current quantity in stock, and the minimum stock quantity 101h indicating the stock quantity by less than which an order must be placed.

Also disposed in the material management window 101 are buttons such as: an Add button 101i for adding material information; an Edit button 101j for editing the contents of registered material information; a Remove button 101k for removing registered material information; an Increase button 1011 and a Decrease button 101m for respectively increasing and decreasing a quantity shown in the stock quantity 101g of the selected material information in usage units of 1; and a Close button 101n for closing the material management window 101.

One type of material information is expediently called a material information record in the present specification. The user suitably adds a material information record after clicking on the Add button 101i. When the quantity shown in the stock quantity 101g is lower than the quantity shown in the minimum stock quantity 101h, the corresponding material information record is shown in red or another such color to urge the user to place an order. Alternatively, a message to encourage an order could also be displayed.

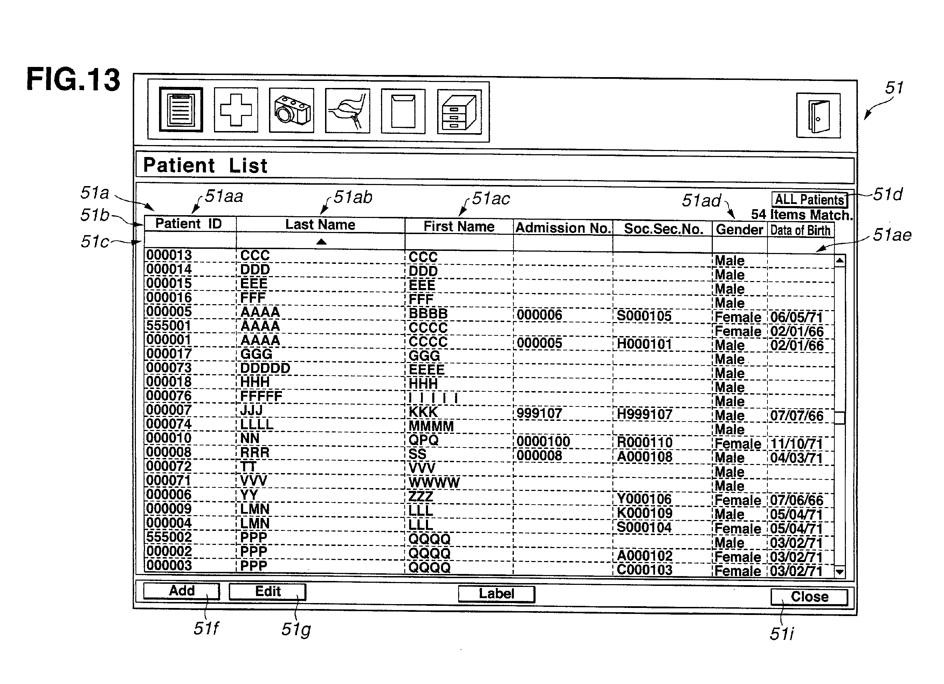

Fig. 13 shows an example of the window display of the patient list window 51.

A patient list display area 51a for displaying a list of patient information is disposed in the patient list window 51.

Patient information corresponding to one patient is expediently called a patient information record in this specification. Patient information records are displayed on and after the third line of the patient list display area 51a, one patient information record corresponding to one line of the patient list display area 51a.

A patient information record displayed in the patient list display area 51a comprises data items such as a patient ID 51aa, the patient's last name 51ab, the patient's first name 51ac, the patient's gender 51ad, and the patient's date of birth 51ae, for example.

Filter buttons 51b corresponding with each data item of the patient information records are disposed in the first line of the patient list display area 51a. Operation of the filter buttons 51b is similar to that of the filter buttons 41d of the schedule list window 41 (refer to Fig. 7).

Sort buttons 51c corresponding with each data item of the patient information records are also disposed in the second line of the patient list display area 51a. Operation of the sort buttons 51c is similar to that of the sort buttons 41e of the schedule list window 41 (refer to Fig. 7).

Upon clicking on an ALL Patients display button 51d, patient information records for all the patients are displayed in the patient list display area 51a.

Disposed at the bottom of the patient list window 51 are: an Add button 51f which is clicked when newly registering a patient; an Edit button 51g which, when clicked after selecting a patient information record in the patient list display area 51a, permits editing of the selected patient information record; and a Close button 51i which, when clicked, closes the patient list window 51 for a return to the window for calling the patient list window 51.

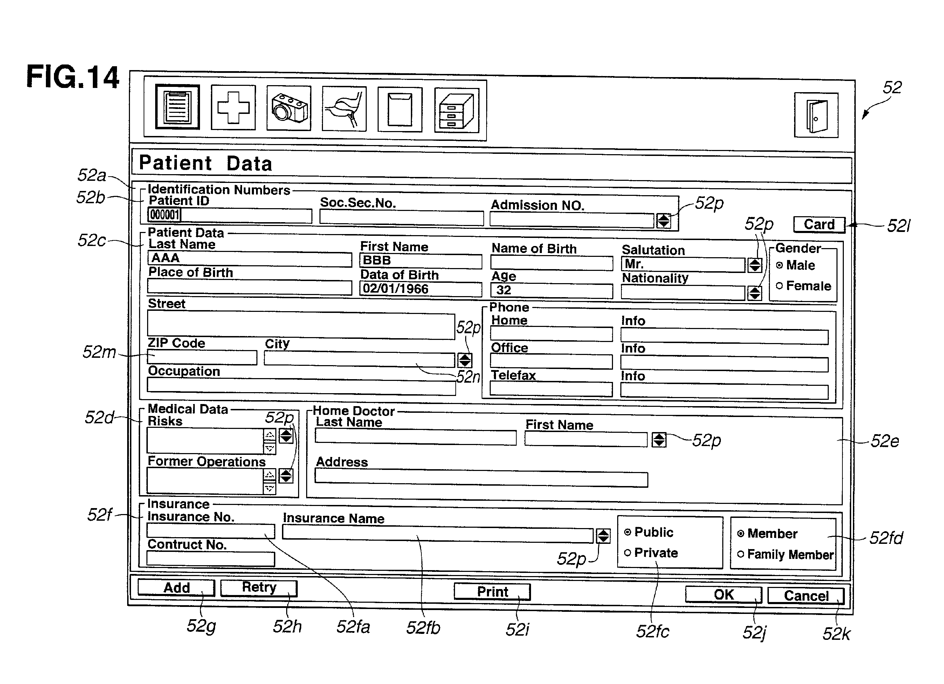

Clicking on the Add button 51f or Edit button 51g calls the patient information editing window 52 shown in Fig. 14.

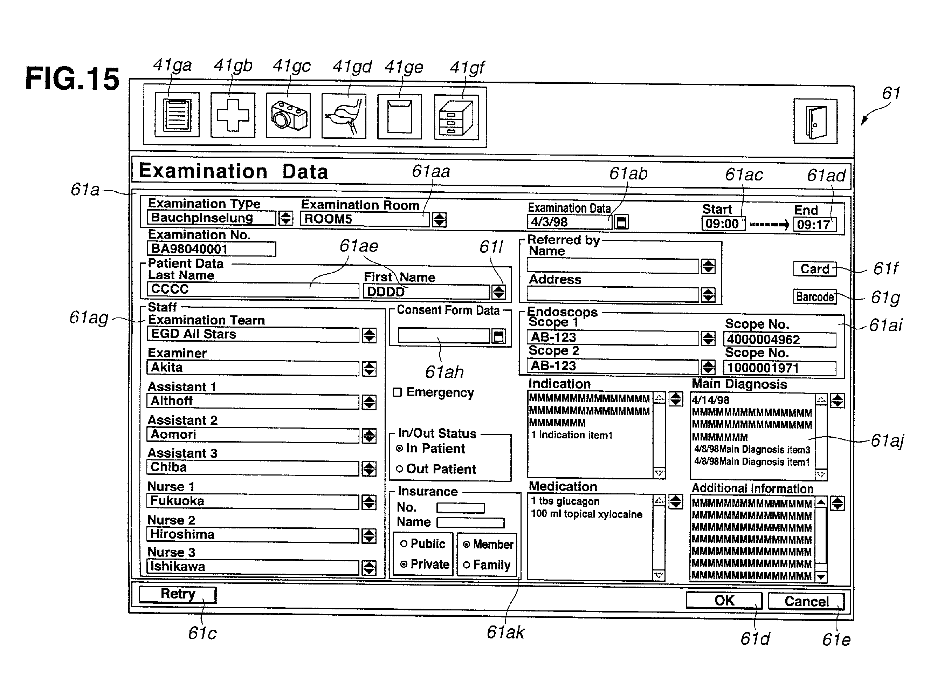

Further, when the patient list window 51 is called from the schedule list window 41 (see Fig. 7), the Close button 51i shown in Fig. 13 is displayed, but when called from the examination information editing window 61 (see Fig. 15) described hereinafter, in place of the Close button 51i, an OK button (not shown) which, when clicked after selecting a patient information record in the patient list window 51, transfers the contents of the selected patient information record to the examination information editing window 61 and closes the patient list window 51, and a Cancel button (not shown) which, when clicked, closes the patient list window 51 without transferring information to the examination information editing window 61, are both displayed.

The patient information editing window 52 shown in Fig. 14 is a window for inputting the contents of a patient information record which is newly registered or editing the contents of existing patient information records.

A region 52a for editing the contents of a patient information record comprises: a region 52b for editing identification numbers such as patient IDs for example; a region 52c for editing the patient's last name, first name, date of birth, birthplace, gender, age, address, home telephone number, office telephone number, office fax number, and so forth; a region 52d for editing a summary of medical information such as a former treatment history with respect to the patient; a region 52e for editing information relating to the patient's home doctor; and a region 52f for editing information relating to the patient's insurance, and the like.

The region 52f has regions disposed therein, namely: a region 52fa for displaying a number for the insurance type (name); a region 52fb for displaying the insurance type; a region 52fc for classifying the insurance type as public or private; and a region 52fd for classifying the form of insurance as that of a member or family member.

Disposed at the bottom of the patient information editing window 52 are: an Add button 52g which, when clicked, newly registers a patient information record of which contents have been entered in the region 52a, initializes the contents entered in the region 52a, and enables subsequent new registration; a Retry button 52h which, when clicked, is capable of retrying editing by restoring the state at the time of opening the patient information editing window 52; a Print button 52i which, when clicked, prints the contents of a patient information record; an OK button 52j which, when clicked, newly adds or updates a patient information record, closes the patient information editing window 52, and restores the patient list window 51; and a Cancel button 52k which, when clicked, closes the patient information editing window 52 and restores the patient list window 51 without newly adding or updating a patient information record. A button 521 for capturing patient information via the card reader 25 or similar is further disposed in a position toward the top of the region 52a.

Upon using the Add button 52g, the patient information record at the time of clicking this button is registered and the contents of the region 52a are initialized without closing the patient information editing window 52, and, consequently, operability for newly registering successively patient information of a plurality of patients is favorable.

A selection list window call button 52p is disposed in a neighboring location to the right of an input field 52m for inputting a ZIP code and an input field 52n for inputting a city name for the patient's address, such fields being in the region 52c for example, in the region 52a. Clicking on the selection list window call button 52p opens selection list windows (not illustrated) that display selection lists for data for entry in corresponding input fields which are the input fields 52m, 52n in this example; and upon selecting data in such select list windows which is to be entered in the input fields 52m, 52n, the selected data is entered in the input fields 52m, 52n. A similar operation is also effected by selection list window call buttons 52p disposed in other locations in the region 52a and by selection list window call buttons disposed in other windows.

Other buttons 52p of the region 52 perform similar operations. In other words, clicking on a button 52p opens selection list windows (not shown) in which selection lists, which are for an insurance company number that is entered in the input field 52fa and for an insurance company name that is entered in the input field 52fb and corresponds with the insurance company number respectively, are displayed. The user selects the insurance company number and insurance company in question from these selection lists. The user also makes an entry to the effect that the selected insurance company is private or public in the region 52fc and enters information on whether the form of patient insurance membership is that of a member or a family member in the region 52fd.

After clicking on the button 521 and feeding a magnetic card for the patient into the card reader 25, patient information is read from the magnetic card and displayed in a region corresponding to the patient information editing window 52. Information such as the patient's first name, the patient's ID, and date of birth for example is registered on the magnetic card. This information is automatically entered in each corresponding input field in the region 52a. In addition to such information, the user can, according to requirements, input patient information and register this information as a patient information record.

The examination information editing window 61 shown in Fig. 15 is a window for newly registering or updating examination information records.

A region 61a for inputting each data item which an examination information record comprises is disposed in the examination information editing window 61.

The region 61a has fields and regions disposed therein, including namely: an input field 61aa for inputting the name of an examination room; a field 61ab for inputting an examination date; a field 61ac for inputting an examination start time; an input field 61ad for inputting an examination end time; input fields 61ae for inputting the patient's last name and first name; a region 61ag comprising an input field for inputting the names of the people in charge of the examination, namely the physician, nurses, and so forth; an input field 61ah for inputting the date on which the examination consent form is received from the patient; a region 61ai comprising input fields for inputting the model numbers and the like of the endoscopes used for the examination; an input field 61aj for inputting treatment results and the like; and a region 61ak for inputting insurance information, and so forth.

Disposed at the bottom of the examination information editing window 61 are, for example: a Retry button 61c which, when clicked, restores information in the region 61a to the state at the time of calling the examination information editing window 61; an OK button 61d which, when clicked, newly registers or updates an examination information record and closes the examination information editing window 61; a Cancel button 61e which, when clicked, closes the examination information editing window 61 without newly registering or updating an examination information record; and disposed in positions toward the top of the examination information editing window 61 are a card button 61f for reading patient information via the card reader 25, and a barcode button 61g for reading a patient ID via the barcode reader 26.

One method for inputting patient information is to click on a button 611 provided alongside the input fields 61ae. Clicking on this button 611 opens the patient list window 51. The user selects the patient in question from among patients displayed, and, upon clicking on an OK button (not shown), the contents of the selected patient information record are displayed in corresponding regions within the examination information editing window 61. When the patient in question has not been registered, the patient information editing window 52 opens automatically and a new patient information record is registered by means of an operation similar to that for registration of a patient information record as described earlier. The user selects the patient information record thus registered and clicks on an OK button not illustrated.

In cases where a magnetic card for the patient can be used, clicking on the card button 61f causes patient information to be read via the card reader 25. When patient information read has already been registered, a search is performed for the patient information record for this patient and the contents of this record are displayed in corresponding regions of the examination information editing window 61.

For example, when the contents recorded on the magnetic card are the patient's name and the patient's ID, the patient's name, which is read from the magnetic card, is displayed in the regions 61ae. Next, a search is performed for the patient information record, based on the patient ID read from the magnetic card, and the insurance information of the corresponding patient information record is read out and displayed in the region 61ak. When patient information which is read has not been registered, the patient information editing window 52 opens automatically. At such time, information read out from the magnetic card, for example information such as the patient's name, the patient's ID, and date of birth, is automatically inputted. The user suitably inputs required information by means of an operation similar to that for registration of a patient information record as described earlier, to thereby register a new patient information record.

When use of a barcode displaying a patient ID is permitted, clicking on the barcode button 61g causes the patient ID to be read via the barcode reader 26. When patient information corresponding to the patient ID thus read has already been registered, a search is performed for the corresponding patient information record based on the patient ID, and the contents of this record are displayed in corresponding regions of the examination information editing window 61.

When patient information corresponding to the patient ID thus read has not been registered, the patient information editing window 52 opens automatically. The user suitably inputs required information to register a new patient information record. The user selects the patient information record thus registered and then clicks on an OK button (not illustrated). The contents of the new patient information are displayed in corresponding regions of the examination information editing window 61.

Patient information which an examination information record comprises is inputted at the time of registering corresponding examination information. For example, when a given patient is registered having family member insurance, upon receiving a given examination, the form of insurance of patient information in this examination information record is registered as that of a family member.

Thereafter, when the same patient is registered having member insurance, upon receiving another examination, the form of insurance of patient information in this examination information record is registered as that of a member. The form of patient insurance registered as described above is displayed in examination state display window fields 41cg and 41ch in the schedule list window 41.

When a patient is specified, examination scheduling is then performed. Scheduling is performed automatically by referring to information on the date and examination room designated by the user as well as to resource schedule records already registered.

The user specifies the patient and specifies the examination type and examination room in the region 61a. Here, the examination rooms can be matched with specified examination types. For example, an upper gastrointestinal tract examination and a lower gastrointestinal tract examination are registered as examination types, examination rooms (1) to (3) being allocated to upper gastrointestinal tract examinations and examination rooms (4) to (6) being allocated to lower gastrointestinal tract examinations. In an operation to specify an examination room, in cases where an upper gastrointestinal tract examination is selected as the examination type, a choice can be made from examination rooms (1) to (3), and in cases where a lower gastrointestinal tract examination is selected as the examination type, a choice can be made from examination rooms (4) to (6).

Standard examination time intervals can be allocated to the examination rooms in consideration of examination type. For example, if such allocation is applied to the example described hereinabove, examination time intervals of 15 minutes and 30 minutes can be allocated to examination rooms (1) to (3) and to examination rooms (4) to (6) respectively. These examination time intervals are desirably such that the time interval for the examination itself includes a time interval for preparation to perform the next examination.

When an examination room and an examination date are designated, the image filing apparatus 2 refers to resource schedules to allocate the examination to a time zone not reserved for another examination, within a designated time interval in which the examination room is available.

For example, in a time zone in which the examination room (1) is available which is from 9:00 until 11:30, when examinations have been reserved from 9:00 until 9:15, and from 9:15 until 9:30 respectively, upon specifying examination room (1) as the next examination information examination room, the examination time interval for this examination is automatically set as being from 9:30 until 9:45.

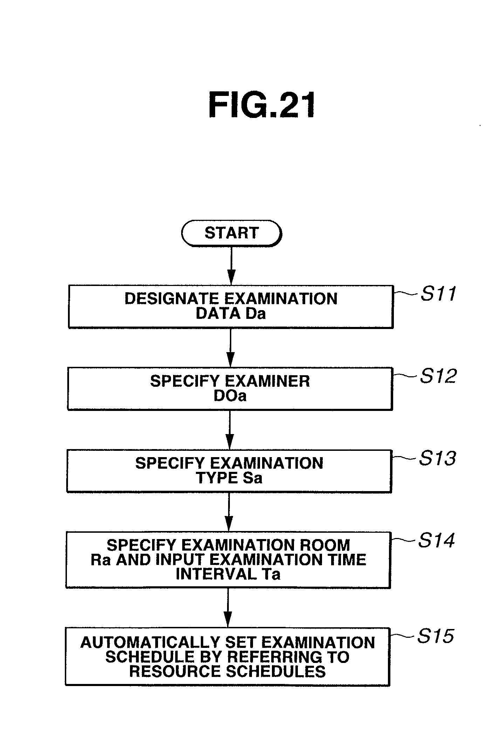

Fig. 21 shows a process to determine an examination schedule in cases where an examined patient is specified.

After specifying a patient in the manner described above, the user designates an examination date Da, as well as an examiner DOa, examination type Sa, and examination room Ra, and inputs an examination time interval Ta required for the examination in the examination room Ra, as shown in steps S11 to S14.

After the information of these steps S11 to S14 is inputted, the CPU 21a of the image filing apparatus 2 refers to the resource schedules as shown in step S15 to automatically set an examination schedule by allocating the examination to a time zone not reserved for another examination, within a designated time interval in which the examination room is available.

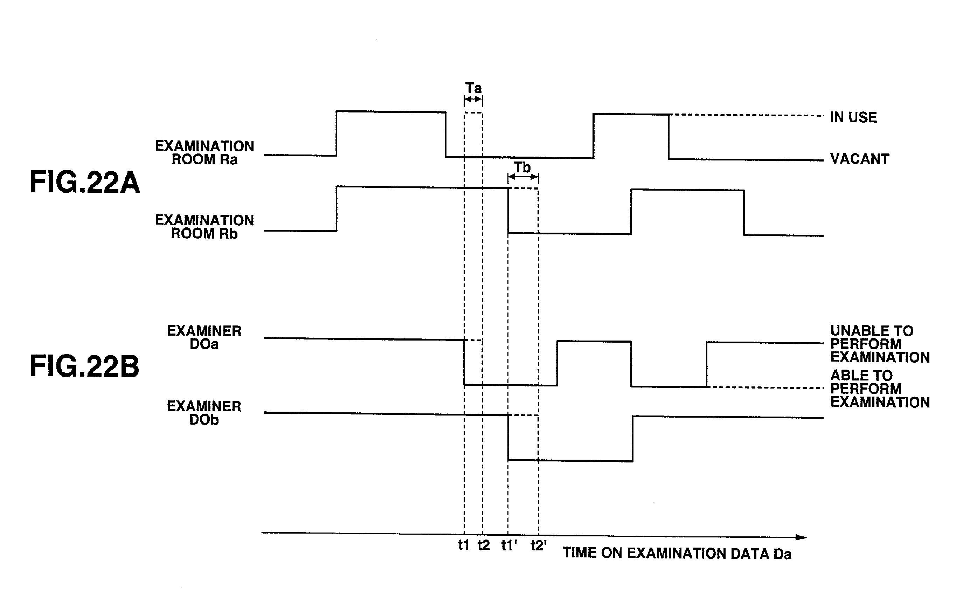

The operation to set an examination schedule by referring to resource schedules in such a case will be described with reference to the schematic diagrams of Figs. 22A and 22B.

Fig. 22A is, for example, for the usage status of the examination room Ra on the examination date Da designated in the resource schedules, and Fig. 22B is for time zones during which the examiner DOa is able to perform an examination (is able to work).

In this case, the CPU 21a of the image filing apparatus 2 also takes into account the examination time interval Ta so as to automatically set, in a time zone in Fig. 22A which is not used for an examination, a time zone having the condition of satisfying a time zone shown in Fig. 22B in which the examiner DOa is able to perform an examination, as indicated by the bold dotted line in Fig. 22A specifically (the examination start time is t1 and the examination end time is t2), for an examination schedule for performing the examination.

Furthermore, the time zone in which the examiner DOa is able to perform an examination changes in accordance with such setting as shown in Fig. 22B. Also, the CPU 21a of the image filing apparatus 2 performs management to incorporate this examination schedule and to update the resource schedules.

Time on the designated examination date Da is plotted on the horizontal axis in Figs. 22A and 22B respectively. Also, on the vertical axis in Fig. 22A, the high level indicates a state in which the examination room is used, and the low level indicates a vacant state of non-usage. Further, on the vertical axis of Fig. 22B, the high level indicates a state in which the examiner DOa is unable to perform an examination because a reservation for an endoscopic examination has been inputted and on account of a personal reservation of this doctor and the like; the low level indicates a state where the examiner DOa is able to perform an examination.

Also, with regard to the resource schedules, the usage status corresponding with examination schedules already registered and so forth is also set for another examination room Rb, as shown in Fig. 22A. Further, time zones during which another examiner DOb is able to perform an examination in correspondence with examination schedules already registered are also set for this examiner. Accordingly, an examination schedule is similarly set when the other examination room Rb is specified.

For example, when, with respect to another patient, the examination date Da is designated; the examination room Rb and examiner Db which are made to correspond so as to perform another examination type (set as Sb) are specified; and an examination time interval Tb, which is set to correspond with the examination type Sb, is inputted, the time zone indicated by the bold dotted lines in Figs. 22A and 22B (the examination start time is t1' and the examination end time is t2') is set, similarly to the case described above.

Setting can also be performed manually to change the examination schedule thus set to another available time zone. In other words, when a change is desired, setting for such a change is possible.

The present embodiment is characterized in that the image filing apparatus 2 refers to information on the date and examination room designated by the user as well as to resource schedules already registered to allocate an examination to a time zone not reserved for another examination, within a time interval in which an examination room is available and which is designated so as to conform with corresponding conditions.

Even in the event of complex conditions that precede the determination of a new examination schedule, it is possible to effectively prevent a situation where schedules are created with conflicting time zones for the same examination room, to thereby permit effective use of available resources.

As described hereinabove, an examination time interval set in this manner can also be changed manually within the available time interval.

Also, when a date for receipt of an examination consent form is entered in the input field 61ah, thereafter the symbol "X" is marked in an examination state display 41cb of a corresponding examination record displayed in the examination list display area 41b of the schedule list window 41.

Next, clicking on the button 41gf opens the material consumption management window 102 into which materials scheduled for use in the examination are inputted.

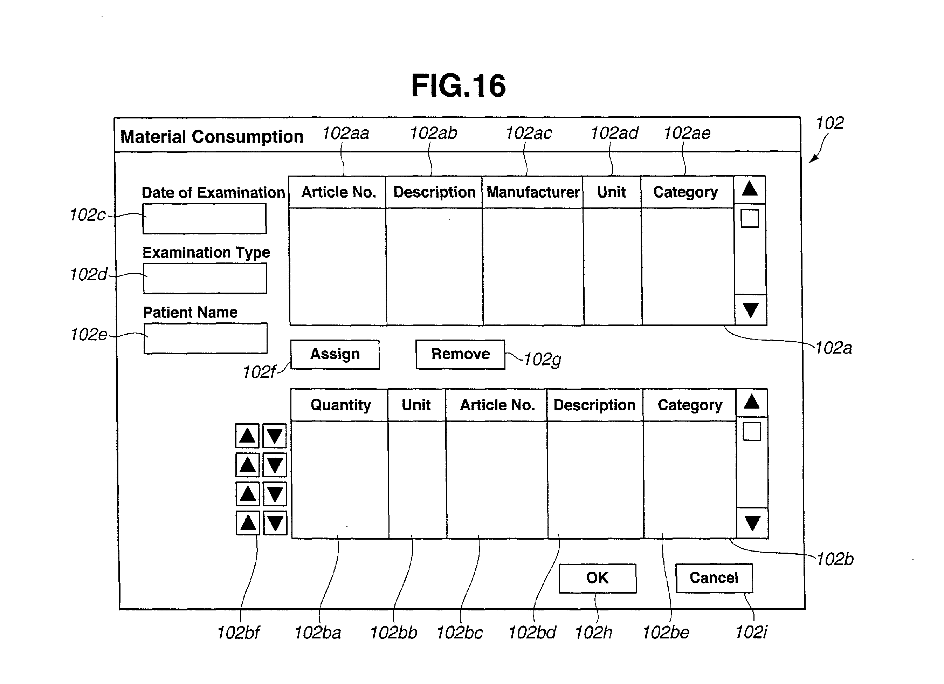

Fig. 16 shows an example of the window display of the material consumption management window 102.

A complete material list 102a and a materials for use list 102b are disposed in the material consumption management window 102. The complete material list 102a comprises items such as an article number 102aa, a description 102ab, a manufacturer 102ac, a unit 102ad indicating an article usage unit, and a category 102ae that indicates whether the article is recyclable or is to be discarded after use for example, the contents of a material information record registered in an material list 101a of the material management window 101 being displayed in the complete material list 102a.

The materials for use list 102b comprises quantities 102ba indicating the quantities of materials used in an examination, units 102bb indicating article usageunits, article numbers 102bc, descriptions 102bd, and categories 102be, and the like. Increase/decrease buttons 102bf, which are for increasing and decreasing in usage units the quantity of a material information record displayed in the materials for use list 102b, are disposed alongside the materials for use list 102b.

Furthermore, disposed in the material consumption management window 102 are: a region 102c for displaying the examination date of the examination in question, a region 102d for displaying the examination type, and a region 102e for displaying the patient's name. Contents entered in the examination information editing window 61 are displayed in these regions.

Further disposed in the material consumption management window 102 are: an Assign button 102f for assigning materials selected in the complete material list 102a to an examination; a button 102g for removing materials assigned to an examination following assignment; a button 102h for saving contents edited in the materials for the materials for use list 102b and closing the material consumption management window 102; and a button 102i for discarding contents edited in the materials for use list 102b and closing the material consumption management window 102.

The user selects materials for use in an examination from the complete material list 102a and adds such materials to the materials for use list 102b by clicking on the Assign button 102f. The user then sets the usage quantity of the materials displayed in the materials for use list 102b by clicking the increase/decrease buttons 102bf. Finally, the user saves the contents displayed in the materials for use list 102b by clicking the button 102h. This information is registered as part of the examination information.

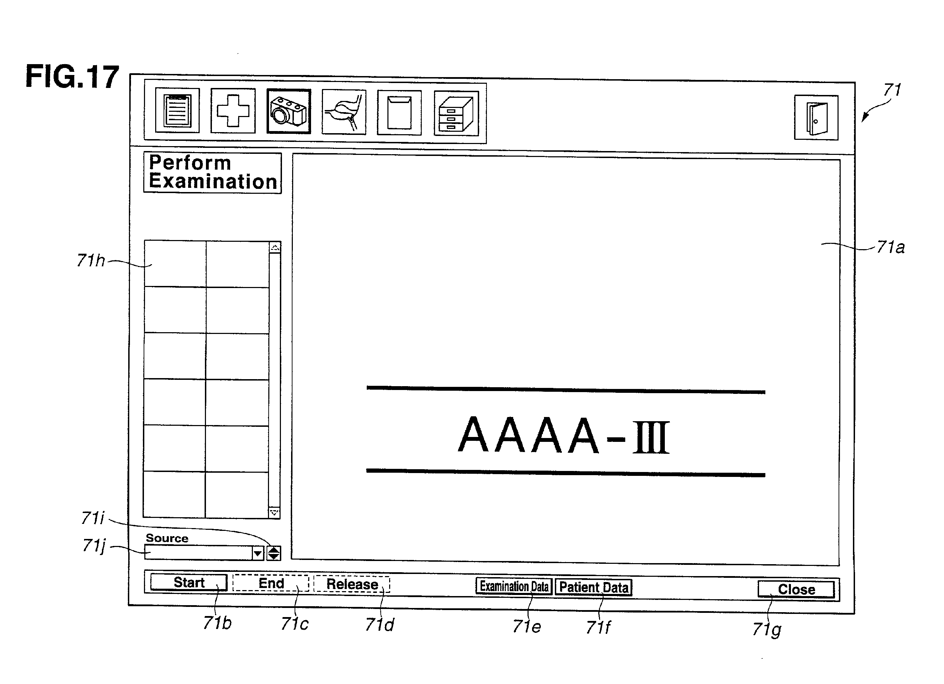

The examination performance window 71 shown in Fig. 17 is a window operated on the image filing apparatus 2 side when performing an examination using the endoscope apparatus 1.

An endoscopic image display area 71a is disposed in the examination performance window 71 and displays images picked up by the endoscope apparatus 1, that is, images that are the same as images displayed on the monitor 15 of the endoscope apparatus 1.

When the user clicks on an examination start button 71b in the examination performance window 71 to start the examination, the image filing apparatus 2 assumes an image capturing state and an endoscopic image is displayed in the endoscopic image display area 71a. The time at this moment is recorded as the examination start time, and an examination end button 71c and an image record button 71d then both assume a state in which the same can be clicked. When the user clicks on the image record button 71d or pushes the endoscope switch 12c, the images at that time are recorded by the image filing apparatus 2. Such images are displayed sequentially in a thumbnail image display area 71h.

When ending an endoscopic examination, the user clicks on the examination end button 71c and the image filing apparatus 2 ends image capturing. The time at this moment is recorded by the image filing apparatus 2 as the examination end time.

When an examination is performed as described above, the symbol "X" is then marked in the examination state display 41cc of the corresponding examination record that is displayed in the operation list display area 41b of the schedule list window 41.

Further, when used in an examination, assigned materials are judged as having been used in an examination by the material consumption management window 102, whereupon the quantities of materials managed by the material management window 101 are automatically reduced by the quantities of the materials used in the examination.

Selecting an examination for which the symbol "X" is marked in the examination state display 41cc in the schedule list window 41, that is, which examination has ended, opens the examination information editing window 61, which allows examination information for this examination to be confirmed. Here, a general user is only able to reference examination information and is not able to change such information.

A user who has been authenticated as an administrator by means of the login window 31 described above is able to modify examination information including patient information. However, in this case also it is necessary to perform a validation operation for modification of information on an examination which has already been executed.

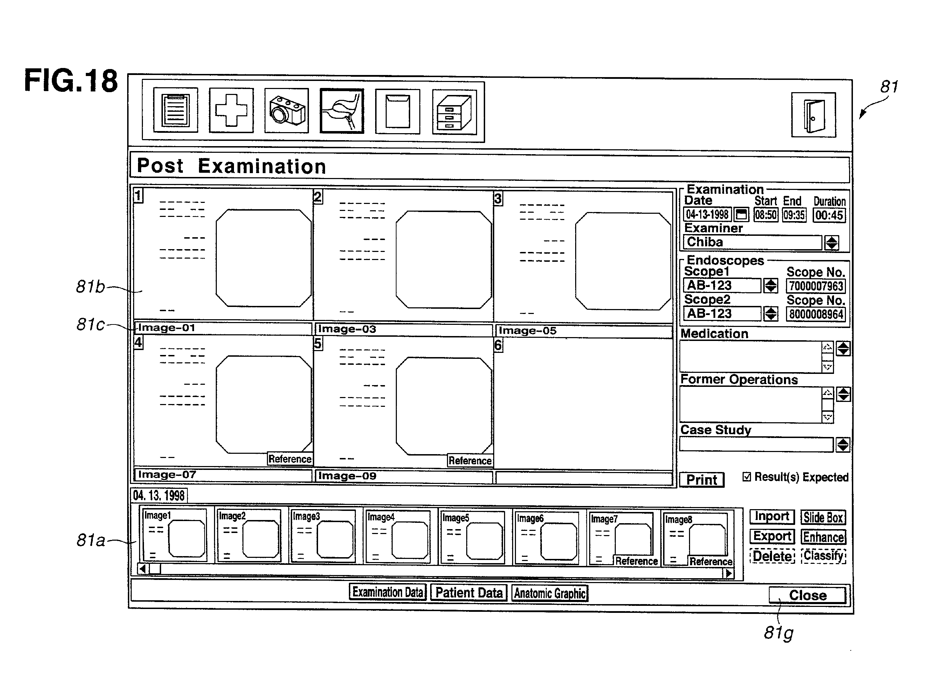

The post-examination processing window 81 shown in Fig. 18 is a window for carrying out post-examination processing that involves the selection of endoscopic images for reference in an examination report from among endoscopic images obtained in performing an examination.

Thumbnail images for all the endoscopic images obtained in performing an examination are displayed in a thumbnail image display area 81a of the post-examination processing window 81.

When the mouse 24 is used to drag one thumbnail image of the thumbnail images displayed in the thumbnail image display area 81a and to drop this thumbnail image in a report image selection area 81b, the endoscopic image that corresponds with this thumbnail image is displayed in the report image selection area 81b. The report image selection area 81b is an area for the selection of endoscopic images for reference in an examination report.

An input field 81c permitting the inputting of notes is disposed below each of images in the report image selection area 81b.

Further, the endoscopic images which are dragged from the thumbnail image display area 81a and dropped in the report image selection area 81b are displayed in the same so as to be sequentially justified from the top left-hand side of the report image selection area 81b.

Part of the examination information is displayed on the right-hand side of the post-examination processing window 81, it being thus possible to edit the examination information.

The user selects the images which are to be used in a report from images picked up in the examination and suitably enters notes on the images thus selected. When the required information has been entered by the user, the user clicks on the Close button 81g disposed at the bottom of the post-examination processing window 81 to thereby end post-examination processing.

Next, the user opens the report creation window 91 to create an examination report. An image display area 91a for displaying a plurality of image thumbnail images selected using the post-examination processing window 81 is disposed in the report creation window 91. While referring to these endoscopic images, the user is thus able to enter an opinion in an opinion structured entry area 91b.

Disposed at the bottom of the report creation window 91 are: a button 91q which, when clicked, switches the window to the report creation window 93; and a Close button 91s which, when clicked, closes the report creation window for a return to the schedule list 41. While referring to images displayed in the image display area 91a, the user suitably enters an opinion in the opinion structured entry area 91b and, once inputting is complete, clicks the button 91q to open the report creation window 93.

Within a report editing area 93a, it is possible to add, delete, edit, move or copy text, or perform other operations, similarly to a publicly known word processor. Also disposed in the report creation window 93 are: a font selection field 93b for setting the pitch of a given character font type; and a plurality of function buttons 93c for calling a variety of functions for editing a document, such as a publicly known spell check function or publicly known character display function for example, the font selection field 93b and function buttons 93c being employed similarly to a publicly known word processor. It is also possible to move, enlarge or reduce the location where images attached to an examination report are placed.

Further, upon clicking on a button 93d provided at the bottom of the report creation window 93, a list window for displaying a list of titles of information which the image filing apparatus 2 comprises is displayed. When an information title is selected in this list window, the information corresponding with the information title thus selected is inserted at the cursor position within the report editing area 93a.

Further disposed at the bottom of the report creation window 93 are: a Print button 93e, which is clicked at the time of printing an examination report; a Save button 93f, which is clicked at the time of saving in "Rich text" format, or similar, for example, an examination report document file in a storage device such as the hard disk 21e or in an external storage device (not shown); a button 93g which, when clicked, switches the window to the report creation window 91; a button 93i which, when clicked, calls a window for a voice recording of an opinion; and a Close button 93j which, when clicked, closes the report creation window for a return to the schedule list 41 (See Fig. 7).

Further, in cases where a voice recording of an opinion is made, a voice recognition device (not shown) is employed. The voice recognition device may also be constituted to perform voice recognition by means of software using the CPU 21a.

In the first embodiment, which has such a constitution and such functions, the endoscopic image filing system 3 is constituted having an endoscope apparatus 1 for performing endoscopic examinations; and an image filing apparatus 2 for recording endoscopic images obtained in the endoscopic examinations by means of the endoscope apparatus 1 as well as information related with these endoscopic examinations, namely patient information and information on examiners (technicians), (endoscopic) examination rooms and examination time intervals, or other information. This endoscopic image filing system 3 comprises: data input means such as the keyboard 23 operated by the user to input data to the image filing apparatus 2; interface means such as the video circuit 21j for inputting endoscopic images from the endoscope apparatus 1 and the communication interface 21i for inputting data related with these endoscopic images; processing means such as the video circuit 21j for performing processing to record or display information obtained by means of the above data input means and interface means; managing means using the CPU 21a or similar for managing schedules of endoscopic examinations performed by means of the endoscope apparatus 1; and display means such as the monitor 22 for displaying processing information of the processing means and management information of the managing means, wherein the managing means automatically creates schedules for the endoscopic examinations on the basis of (and in a broader application by means of at least either basis of) conditions such as time zones in which examination rooms are available and time zones in which examiners are able to work, these conditions being preset via the data input means, and on the basis of information such as information on dates and times and on examination rooms for performing the endoscopic examinations, such information being inputted via the data input means at the time an endoscopic examination is reserved or received.

Therefore, according to the present embodiment, it is possible to reduce the user labor that results when the user refers to the above-described conditions and information in order to manually put together a schedule that conforms with such conditions and information, and to effectively utilize resources such as examination rooms.

It is further possible to effectively use supplied resources and to avoid a situation where there is a conflict between schedules, even under complex conditions.

Input errors can also be reduced through simplification by adopting the card reader 25 or similar as the input means for information on patients receiving endoscopic examinations.

In addition, the management of materials used in endoscopic examinations can also be performed automatically, orders for materials being placed at the appropriate time to permit the provision of suchmaterials for endoscopic examinations. In other words, in the present embodiment, management is carried out by integrating resources such as consumables required for endoscopic examinations with resources such as examination rooms, and hence it is possible to reliably prevent the occurrence of a situation where there is a shortage of the resources required for endoscopic examinations, which would hinder endoscopic examinations, and it is possible to provide an environment for performing endoscopic examinations smoothly.

### (Second embodiment)

A second embodiment of the present invention will be described next. The present embodiment has a constitution similar to that of the first embodiment, but the endoscopic examination schedule method is different.

The user enters patient information by means of an operation similar to that of the first embodiment using the examination information editing window 61 shown in Fig. 15.

Next, the user specifies an examiner in the region 61ag. Here, each examiner can be linked with an examination type. That is, upon first specifying the examiner, the examination type is specified automatically. Also, once the examination type has been first specified, when an examiner is specified, a choice can be made from examiners who are linked with the examination type.

When examiners are also linked with examination rooms, an examination room may also be automatically specified when an examiner is specified.

Next, when an examination date is entered in the region 61a, the endoscopic image filing apparatus 2 specifies times that satisfy an examination time interval required for the specified examination type, on a designated examination date, in a time zone in which a specified examiner can work, and in a time zone in which a specified examination room can be used, and displays the specified examination start time and examination end time in the regions 61ac and 61ad.

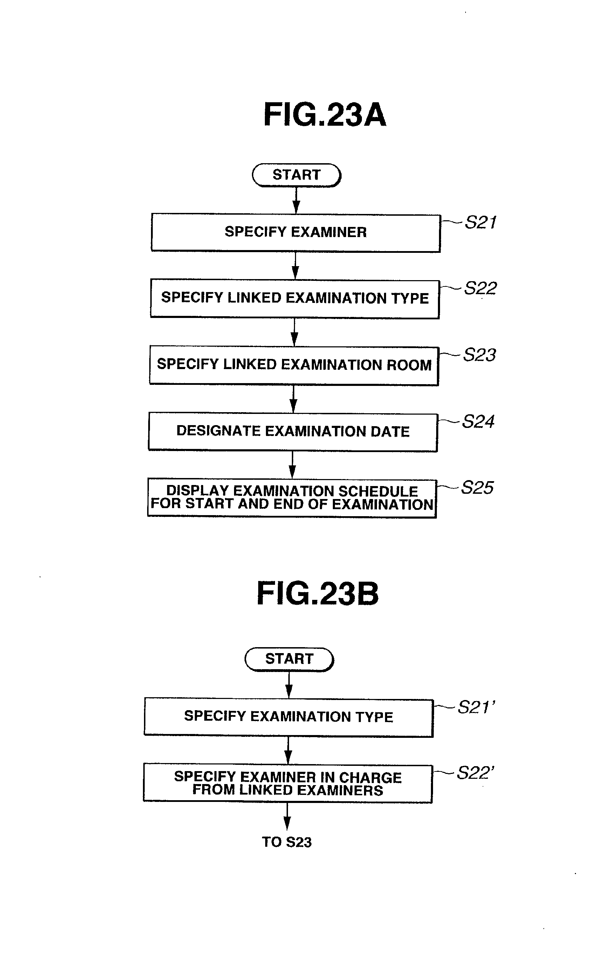

Figs. 23A and 23B illustrate processes whereby an examination schedule is determined according to the present embodiment.

After specifying a patient, the user specifies an examiner in step S21. Thus, the examination type which is linked with the examiner is specified, as shown in step S22.

The examination room linked with the examiner is also specified, as shown in step S23.

Alternatively, it is also possible to first specify an examination type (step S21'), and then specify an examiner (person in charge) from a plurality of examiners linked with this examination type (step S22'), as shown in Fig. 22B.

The examination date is entered in the next step S24. Thus, an examination schedule is displayed by setting an examination start time and examination end time in accordance with this information, as shown in step S25.

In other words, the endoscopic image filing apparatus 2 refers to this information to specify times that satisfy an examination time interval required for the specified examination type, on a designated examination date, in a time zone in which a specified examiner can work, and in a time zone in which a specified examination room can be used, and displays the specified examination start time and examination end time in the regions 61ac and 61ad respectively, whereby an examination schedule is automatically set.

According to the present embodiment, user labor involved in required data input operations is reduced still further. Otherwise, this embodiment has effects similar to those of the first embodiment.

Further, the present invention is not limited to or by the embodiments above, various modifications being possible within the scope of the invention without departing from the spirit thereof.

For example, the mouse 24 is not limited to a mouse, but rather may also be constituted by a trackball or other pointing device.

Also, for example, the endoscope 12 is not limited to an electronic endoscope constituted having image pickup means 12a provided in the tip of the inserting portion of the endoscope, but rather may also be an endoscope constituted using an image guide fiber (not illustrated) or similar to optically transmit images of the examination site 11 from the tip of the inserting portion to image pickup means (not illustrated) provided on the proximal end of the endoscope.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. Endoscopic image filing system, comprising:
an information input unit operated for inputting information;
an interface section for inputting endoscopic images from an endoscope apparatus and information related with the endoscopic images;
processing circuit for processing information obtained using said information input unit and interface section;
managing apparatus for managing schedules for endoscopic examinations performed using said endoscope apparatus; and
display device for displaying processing information of said processing means and management information of said managing means,
**characterized in that** said managing apparatus comprise schedule creating means for automatically creating said endoscopic examination schedules by means of at least either setting conditions preset via said information input unit, or inputting information inputted via said information input unit when said endoscopic examinations are received.

2. The endoscopic image filing system according to Claim 1, wherein said setting conditions comprise at least any of: information on time zones in which one or more endoscopic examination rooms is/are available; information on time intervals required for examinations with respect to one or more endoscopic examination types; and information on time zones in which one or more examiners performing said endoscopic examinations can work; and, wherein said inputting information comprises at least any of: information on the date and time for performing said endoscopic examinations; information on said endoscopic examination types; information on said endoscopic examination rooms which are used; and information on examiners performing said endoscopic examinations.

3. The endoscopic image filing system according to Claim 1, wherein said managing apparatus further create said endoscopic examination schedules such that one endoscopic examination type is allocated to each of said endoscopic examination rooms.

4. The endoscopic image filing system according to Claim 1, wherein schedules managed by said managing means are resource schedules, and said schedule creating section create said endoscopic examination schedules such that conformity exists between said inputted information and information such as information on time zones that are open in said resource schedules.

5. The endoscopic image filing system according to Claim 1, wherein, when said endoscopic examination schedules are created by said schedule creating means, information of these schedules is automatically reflected on schedules managed by said managing means.

6. The endoscopic image filing system according to Claim 1, wherein saidmanagingmeans centrally manage information comprising endoscopic images from said endoscope apparatus, schedule information of schedules for performing said endoscopic examinations, and material information such as stock quantities of materials used in said endoscopic examinations.

7. Endoscopic image filing system management method, comprising:
an information inputting step of inputting information by operating an information input unit;
an external inputting step of inputting endoscopic images from an endoscope apparatus and information related with these endoscopic images via an interface section;
a processing step of processing information obtained in said information inputting step and external inputting step;
a managing step of managing schedules for endoscopic examinations performed using said endoscope apparatus; and
a displaying step of displaying, on a display section, processing information processed in said processing step and management information managed in said managing step,
wherein said managing step further comprises a schedule creating step of automatically creating said endoscopic examination schedules by means of at least either preset conditions inputted via said information input unit, or inputting information inputted via said information input unit when said endoscopic examination application is received.

8. The endoscopic image filing system management method according to Claim 7, wherein said preset conditions comprise at least any of: information on time zones in which one or more endoscopic examination rooms is/are available; information on time intervals required for examinations with respect to one or more endoscopic examination types; and information on time zones in which one or more examiners performing said endoscopic examinations can work, and, wherein said inputting information thus inputted comprises at least any of: information on the date and time for performing said endoscopic examinations; information on types of said endoscopic examinations; information on said endoscopic examination rooms which are used; and information on examiners performing said endoscopic examinations.

9. The endoscopic image filing system management method according to Claim 7, wherein said schedule creating step further creates said endoscopic examination schedules such that one endoscopic examination type is allocated to each of said endoscopic examination rooms.

10. The endoscopic image filing system management method according to Claim 7, wherein, when said endoscopic examination schedules are created by means of said schedule creating step, schedules managed by means of said managing step are read as management schedule information and such that said endoscopic examination schedules conform with said management schedules.
